# EUROPEAN PATENT APPLICATION

(11) **EP 4 424 296 A1**
(43) Date of publication of application: **04.09.2024**
(21) Application number: 22886930.1
(22) Date of filing: 24.10.2022
(51) Int. Cl.: A61K 8/06, A61K 8/34, A61K 8/362, A61K 8/365, A61K 8/41, A61K 8/46, A61K 8/73, A61K 8/81, A61Q 5/00, A61Q 5/12

(54) **METHOD FOR PRODUCING EMULSION COMPOSITION**

(30) Priority: 25.10.2021 JP 2021174037; 27.07.2022 JP 2022119762
(71) Applicant: Kao Corporation, Tokyo 103-8210 (JP)
(72) Inventor: MIYOSHI, Eisuke, Tokyo 131-8501 (JP); UENO, Shiori, Tokyo 131-8501 (JP); SARUKAWA, Yuri, Tokyo 131-8501 (JP); KAWAMOTO, Koichi, Tokyo 131-8501 (JP)
(74) Representative: Hoffmann Eitle
(86) International application number: PCT/JP2022/039464
(87) International publication number: WO 2023/074603

(57) **Abstract**

Provided is a method for producing an emulsion composition containing: component (A): one or more polymers selected from the group consisting of cationic polymers and amphoteric polymers; component (B): an anionic polymer; component (C): an organic acid; and component (D): water, the method including the following step (1) and the following step (2-1) or step (2-2), in which a total blending amount of the component (A) and the component (B) in the emulsion composition is less than 1.0% by mass based on 100% by mass of the total amount of the emulsion composition:
step (1): a step for preparing a mixture A containing the components (A) to (D);
step (2-1): a step for preparing an emulsion (I) by mixing the mixture A with an oil phase component containing an emulsion forming component;
step (2-2): a step for preparing an emulsion (II) by mixing an oil phase component containing an emulsion forming component with an aqueous phase component other than the mixture A, followed by mixing with the mixture A.

## Description

### Field of the Invention

The present invention relates to a method for producing an emulsion composition.

### Background of the Invention

Cosmetic compositions containing a cationic polymer and an anionic polymer are known. For example, JP 53-139734 A (PTL 1) describes that by using a composition for treating a keratin material, which is characterized by containing at least one anionic polymer and at least one cationic polymer in a solvent medium, the anionic polymer can be fixed to a keratin material that can particularly include hair, skin or nails. In addition, it is assumed that a complex formed by an interaction between the anionic polymer and the cationic polymer is related to the reason why the above-described effect is obtained.

As a composition containing a complex formed of a cationic polymer and an anionic polymer (hereinafter also referred to as a "polyion complex"), a composition in the form of an oil-in-water type or water-in-oil type emulsion composition is known. In general, the improvement of stability with time is a problem in an emulsion composition, and a stable emulsion composition or a method for producing a stable emulsion composition has been studied.

For example, JP 11-116445 A (PTL 2) discloses that a hair treatment agent composition in a stable emulsified state in which a polymer supplied in the form of a powder is blended can be easily produced by producing a hair treatment agent composition by a step of emulsifying oil and moisture with a surfactant, a step of dissolving a powder-like cationic polymer and a powder-like anionic polymer in water, and a step of mixing these.

JP 2019-1728 A (PTL 3) discloses, as a stable composition containing polyion complex particles having various cosmetic functions, a composition containing: (a) at least one particle containing at least one cationic polymer and at least one amphoteric polymer, at least one anionic polymer and at least one amphoteric polymer, or at least one amphoteric polymer, and at least one non-polymeric acid having two or more pKa values or a salt thereof or at least one non-polymeric base having two or more pKb values or a salt thereof; and (b) at least one physiologically acceptable volatile medium.

### Summary of the Invention

The present invention relates to the following.

A method for producing an emulsion composition containing the following components:
component (A): one or more polymers selected from the group consisting of cationic polymers and amphoteric polymers;
component (B): an anionic polymer;
component (C): an organic acid; and
component (D): water,
the method including the following step (1) and the following step (2-1) or step (2-2),
in which a total blending amount of the component (A) and the component (B) in the emulsion composition is less than 1.0% by mass based on 100% by mass of the total amount of the emulsion composition:
   step (1): a step for preparing a mixture A containing the components (A) to (D);
   step (2-1): a step for preparing an emulsion (I) by mixing the mixture A with an oil phase component containing an emulsion forming component;
   step (2-2): a step for preparing an emulsion (II) by mixing an oil phase component containing an emulsion forming component with an aqueous phase component other than the mixture A, followed by mixing with the mixture A.

### Detailed Description of the Invention

As disclosed in PTLs 1 to 3, a highly stable emulsion composition containing a polyion complex and a method for producing the same have been studied. However, there is still room for improvement in performance when an emulsion composition containing a polyion complex is used as a cosmetic composition.

For example, in a hair conditioner which is a type of hair cosmetic composition, it is required that the feel when applied to the hair is good, and that the hair after treatment does not spread even under high humidity conditions and has excellent moisture resistance.

As a method for improving the feel of the hair at the time of application to the hair or the feel of the hair after treatment, a method of blending a cationic surfactant, a higher alcohol, or the like into a hair conditioner is generally known, but an emulsion composition in which these components are blended into a composition containing a polyion complex has a problem of low stability. In addition, regarding the suppression of spread of the hair under high humidity conditions, no particular effect has been obtained in the prior art.

An object of the present invention is to provide a method for producing an emulsion composition which contains a polyion complex, has high stability, and has a good feel at the time of application to the hair and can suppress the spread of the hair after treatment under high humidity conditions when used as a hair cosmetic composition.

The present inventors have found that the above-mentioned problems can be solved by preparing a mixture by mixing one or more polymers selected from the group consisting of a cationic polymer and an amphoteric polymer, an anionic polymer, an organic acid, and water, and producing an emulsion composition by a predetermined step using the mixture.

According to the present invention, it is possible to provide a method for producing an emulsion composition which contains a polyion complex, has high stability, and has a good feel at the time of application to the hair and can suppress the spread of the hair after treatment under high humidity conditions when used as a hair cosmetic composition.

### [Method for Producing Emulsion Composition]

The method for producing an emulsion composition of the present invention (hereinafter, also simply referred to as "the production method of the present invention") is a method for producing an emulsion composition containing:
component (A): one or more polymers selected from the group consisting of cationic polymers and amphoteric polymers;
component (B): an anionic polymer;
component (C): an organic acid; and
component (D): water,
the method including the following step (1) and the following step (2-1) or step (2-2),
in which a total blending amount of the component (A) and the component (B) in the emulsion composition is less than 1.0% by mass based on 100% by mass of the total amount of the emulsion composition:
   step (1): a step for preparing a mixture A containing the components (A) to (D);
   step (2-1): a step for preparing an emulsion (I) by mixing the mixture A with an oil phase component containing an emulsion forming component;
   step (2-2): a step for preparing an emulsion (II) by mixing an oil phase component containing an emulsion forming component with an aqueous phase component other than the mixture A, followed by mixing with the mixture A.

By having the above-described constitution, the production method of the present invention can produce an emulsion composition which contains a polyion complex, has high stability, and has a good feel at the time of application to the hair and can suppress the spread of the hair under high humidity conditions when used as a hair cosmetic composition. Hereinafter, the steps (2-1) and (2-2) may be collectively referred to as "step (2)".

### <Definitions>

In the description herein, the "emulsion forming component" means an oily component for forming an emulsion contained in the emulsion composition obtained by the production method of the present invention.

In the description herein, the "aqueous phase component" means a component constituting the aqueous phase in the production step of the emulsion composition, and the "oil phase component" means a component constituting the oil phase in the production step of the emulsion composition.

Although the reason why the production method of the present invention exhibits the above effect is not clear, it is considered as follows.

The emulsion composition produced by the present invention contains a polyion complex formed from the component (A) and the component (B).

The polyion complex is an aggregate formed by a chemical electrostatic interaction between the component (A) and the component (B). The polyion complex is poorly watersoluble, and in the emulsion composition of the present invention, it is present in a state of being dispersed in an aqueous phase. It is considered that a hydrophobic film can be formed by applying an emulsion composition containing the polyion complex to the surface of skin, hair, or the like, and by the formation of the hydrophobic film, the feel at the time of application to the hair is improved, the moisture resistance is improved, and the spread of the hair under high humidity conditions can be suppressed.

In the production method of the present invention, in the step (1), the components (A) to (D) are mixed in advance to obtain a mixture A (aqueous phase component) containing a polyion complex formed from the component (A) and the component (B). Here, by dispersing the component (A) and the component (B) in water in the presence of the organic acid which is the component (C), the obtained polyion complex is stably dispersed and formed even in the emulsion composition. In addition, by setting the total blending amount of the component (A) and the component (B) to less than 1.0% by mass based on 100% by mass of the total amount of the obtained emulsion composition, the stability of the emulsion composition is further improved.

Further, in the present invention, after preparing the mixture A containing the polyion complex in the step (1), the mixture A and the oil phase component containing the emulsion forming component and the aqueous phase component other than the mixture A are mixed in a predetermined order in the step (2-1) or the step (2-2). It is considered that by going through this step, even when other aqueous phase components and oil phase components are blended in the step (2) to form an emulsion, the polyion complex formed in the step (1) is dispersed in a good state without aggregation, separation, etc., and thus the stability of the emulsion composition is improved. When the emulsion composition is used as a hair cosmetic composition such as a hair conditioner, it is considered that the polyion complex emulsified and dispersed in a good state adheres to the hair uniformly, and the feel at the time of application to the hair and the effect of suppressing the spread of the hair under high humidity conditions are improved.

As described later, by adjusting the amount of the component (C) used for the preparation of the mixture A in the step (1) to a predetermined range, the obtained polyion complex is in a state of a suspension dispersed without being solubilized in the mixture A. Then, it is considered that in a case that the mixture A exhibiting a suspension state is used in the step (2-1) or (2-2), even when other aqueous phase components and oil phase components are blended in the step (2), the state of the stable polyion complex is easily maintained in the obtained emulsion composition, and the stability of the emulsion composition, the feel at the time of application to the hair, and the effect of suppressing the spread of the hair under high humidity conditions are further improved.

The emulsion composition obtained by the production method of the present invention is preferably an oil-in-water emulsion composition from the viewpoint of stability and from the viewpoint of improving the feel in use.

The emulsion composition is suitably used as a cosmetic composition such as a hair cosmetic composition and a skin cosmetic composition. Among these, from the viewpoints of good feel at the time of application to the hair and the effect of suppressing the spread of the hair under high humidity conditions, a hair cosmetic composition is preferred.

Examples of the hair cosmetic composition include a hair shampoo, a hair rinse, a hair conditioner, a hair treatment (including a type that is not washed away), and a hair styling agent. Among these, from the viewpoint of the effectiveness of the effect of the present invention, a hair conditioner, a hair treatment, or a hair styling agent is preferable.

Hereinafter, each component to be blended in the emulsion composition will be described.

### (Component (A): One or more polymers selected from the group consisting of cationic polymers and amphoteric polymers)

The component (A) is one or more polymers selected from the group consisting of cationic polymers and amphoteric polymers, which can form a polyion complex by interaction with the component (B).

The cationic polymer as the component (A) preferably means a polymer having a cationic group and substantially having no anionic group and amphoteric group. The phrase "substantially having no anionic group and amphoteric group" means that the molar amount of the anionic group and the amphoteric group with respect to the cationic group is preferably 0.1% or less.

The amphoteric polymer as the component (A) is preferably a polymer having a cationic group and an anionic group, and the pH of a 1% aqueous solution of the polymer at 25°C is less than 5.1, and the polymer is positively charged as the total charge of the polymer. The pH can be measured with a pH meter.

The cationic group in the description herein is a cationic group or a group that can be ionized to become a cationic group, and specific examples thereof include a primary amino group, a secondary amino group, a tertiary amino group, and a quaternary ammonium group.

The anionic group is an anion group or a group that can be ionized to form an anion group, and specific examples thereof include one or more selected from the group consisting of acidic groups such as a carboxy group, a sulfonic acid group, and a phosphoric acid group, preferably one or more selected from the group consisting of a carboxy group and a sulfonic acid group, and more preferably a carboxy group. At least a part of the anionic groups may be neutralized and in the form of a salt.

The cationic charge density of the component (A) is preferably 0.1 meq/g or more, more preferably 0.5 meq/g or more, still more preferably 1.0 meq/g or more, and even more preferably 2.0 meq/g or more, from the viewpoint of facilitating the formation of the polyion complex by interaction with the component (B), and is preferably 10 meq/g or less, more preferably 8.0 meq/g or less, and still more preferably 7.0 meq/g or less, from the viewpoint of stability of the emulsion composition. Thus, the cationic charge density of the component (A) is preferably 0.1 meq/g or more and 10 meq/g or less, more preferably 0.5 meq/g or more and 8.0 meq/g or less, still more preferably 1.0 meq/g or more and 7.0 meq/g or less, and even more preferably 2.0 meq/g or more and 7.0 meq/g or less.

The cationic charge density of the component (A) can be calculated from (the number of moles of cationic groups contained per 1 g of polymer) × 1000 (meq/g).

The emulsion composition may use two or more polymers as the component (A), and in this case, the cationic charge density of the component (A) is obtained by calculating the weighted average from the cationic charge density and the blending amount of each polymer.

The weight average molecular weight (Mw) of the component (A) is preferably 5,000 or more, more preferably 8,000 or more, and is preferably 2,000,000 or less, more preferably 1,500,000 or less from the viewpoints of facilitating the formation of the polyion complex, stability of the emulsion composition, improvement in feel at the time of application to the hair when the emulsion composition is used as a hair cosmetic composition, and suppression of spread of the hair under high humidity conditions. Thus, the weight average molecular weight (Mw) of the component (A) is preferably 5,000 or more and 2,000,000 or less, and more preferably 8,000 or more and 1,500,000 or less.

The weight average molecular weight of the component (A) can be measured by gel permeation chromatography (GPC).

Specific examples of the polymer used as the component (A) include a cationized guar gum, a cationized tara gum, a cationized locust bean gum, a cationic starch, a cationized cellulose, a cationized hydroxyalkyl cellulose, a cationized polyvinyl alcohol, a polyethyleneimine, a quaternized dialkylaminoalkyl (meth)acrylate polymer, a diallyl quaternized ammonium salt polymer, a methacrylamidopropyltrimethylammonium salt polymer, a methacryloyl ethyltrimethylammonium salt polymer, a vinyl imidazolium trichloride-vinylpyrrolidone copolymer (polyquaternium-16), a vinyl pyrrolidone-alkylamino(meth)acrylate copolymer, a vinyl pyrrolidone-alkylamino(meth)acrylate-vinyl caprolactam copolymer, an alkyl acrylamide-(meth)acrylate-alkylamino alkylacrylamide-polyethylene glycol (meth)acrylate copolymer, and an adipic acid-dimethylaminohydroxypropyl ethylenetriamine copolymer. One, or two or more of these can be used.

Among the above, examples of the cationized hydroxyalkyl cellulose include cationized hydroxyethyl cellulose and cationized hydroxypropyl cellulose, and examples of the cationized hydroxyethyl cellulose include o-[2-hydroxy-3-(trimethylammonio)propyl]hydroxyethyl cellulose chloride (polyquaternium-10).

Examples of the quaternized dialkylaminoalkyl (meth)acrylate polymer include a vinylpyrrolidone-N,N-dimethylaminoethyl methacrylate diethyl sulfate copolymer (polyquaternium-11), and an N,N-dimethylaminoethyl methacrylate diethyl sulphate-N,N-dimethylacrylamide-polyethylene glycol dimethacrylate copolymer (polyquaternium-52).

Examples of the diallyl quaternized ammonium salt polymer include a dimethyldiallylammonium chloride polymer (polyquaternium-6), a dimethyldiallylammonium chloride-acrylic acid copolymer (polyquaternium-22), a diallyldimethylammonium chloride-acrylamide copolymer (polyquaternium-7), and an acrylamide-acrylic acid-dimethyldiallylammonium chloride copolymer (polyquaternium-39).

Examples of the methacrylamidopropyltrimethyl ammonium salt polymer include a methacrylamidopropyltrimethyl ammonium chloride polymer, a vinylpyrrolidone-methacrylamidopropyltrimethyl ammonium chloride copolymer, an acrylic acid-methyl acrylate-methacrylamidopropyltrimethyl ammonium chloride copolymer (polyquaternium-47), and an acrylic acid-acrylamide-methacrylamidopropyltrimethyl ammonium chloride copolymer (polyquaternium-53).

Examples of the methacryloyl ethyl trimethyl ammonium salt polymer include a methacryloyl ethyl trimethyl ammonium chloride polymer (polyquaternium-37), a methacryloyl ethyl dimethyl betaine-methacryloyl ethyl trimethyl ammonium chloride-methoxypolyethylene glycol methacrylate copolymer (polyquaternium-49), and a methacryloyl ethyl dimethyl betaine-methacryloyl ethyl trimethyl ammonium chloride-2-hydroxyethyl methacrylate copolymer (polyquaternium-48).

The component (A) can be used alone or in combination of two or more thereof. Among the above, from the viewpoints of facilitating the formation of the polyion complex, stability of the emulsion composition, and improvement in feel at the time of application to the hair and suppression of spread of the hair under high humidity conditions when the emulsion composition is used as a hair cosmetic composition, the component (A) is at least a polymer having a cationic group.

From the viewpoints of stability of the emulsion composition, and improvement in feel at the time of application to the hair and suppression of spread of the hair under high humidity conditions when the emulsion composition is used as a hair cosmetic composition, the component (A) is more preferably at least one selected from the group consisting of a quaternized dialkylaminoalkyl (meth)acrylate polymer, a diallyl quaternized ammonium salt polymer, a methacrylamidopropyl trimethylammonium salt polymer, a methacryloyl ethyl trimethylammonium salt polymer, and a vinyl imidazolium trichloride-vinyl pyrrolidone copolymer (polyquaternium-16), still more preferably one or more selected from the group consisting of a diallyl quaternized ammonium salt polymer, a methacrylamidopropyl trimethylammonium salt polymer, and a vinyl imidazolium trichloride-vinyl pyrrolidone copolymer (polyquaternium-16), even more preferably one or more selected from the group consisting of a dimethyldiallylammonium chloride polymer (polyquaternium-6), a dimethyldiallylammonium chloride-acrylic acid copolymer (polyquaternium-22), an acrylamide-acrylic acid-dimethyldiallylammonium chloride copolymer (polyquaternium-39), an acrylic acid-methyl acrylate-methacrylamidopropyl trimethylammonium chloride copolymer (polyquaternium-47), and a vinyl imidazolium trichloride-vinyl pyrrolidone copolymer (polyquaternium-16), and even more preferably one or more selected from the group consisting of a dimethyldiallylammonium chloride polymer (polyquaternium-6), an acrylamide-acrylic acid-dimethyldiallylammonium chloride copolymer (polyquaternium-39), and a vinyl imidazolium trichloride-vinyl pyrrolidone copolymer (polyquaternium-16).

Among the above, the preferable ranges of the cationic charge density and the weight average molecular weight of the following polymers are preferably as follows from the viewpoint of facilitating the formation of the polyion complex, and from the viewpoints of stability of the emulsion composition, and improvement in feel at the time of application to the hair and suppression of spread of the hair under high humidity conditions when the emulsion composition is used as a hair cosmetic composition.

The cationic charge density of the dimethyldiallylammonium chloride polymer (polyquaternium-6) is even more preferably 2.0 meq/g or more, and even more preferably 2.5 meq/g or more, and is even more preferably 6.5 meq/g or less. The weight average molecular weight is still more preferably 10,000 or more and 200,000 or less.

The cationic charge density of the dimethyldiallylammonium chloride-acrylic acid copolymer (polyquaternium-22) is even more preferably 2.0 meq/g or more, even more preferably 3.0 meq/g or more, and even more preferably 4.0 meq/g or more, and is even more preferably 6.5 meq/g or less, and even more preferably 6.0 meq/g or less. The weight average molecular weight is still more preferably 20,000 or more, even more preferably 50,000 or more, even more preferably 100,000 or more, even more preferably 200,000 or more, and even more preferably 300,000 or more, and is still more preferably 1,000,000 or less, even more preferably 600,000 or less, and even more preferably 500,000 or less.

The cationic charge density of the acrylamide-acrylic acid-dimethyldiallylammonium chloride copolymer (polyquaternium-39) is even more preferably 2.0 meq/g or more, and even more preferably 2.5 meq/g or more, and is even more preferably 6.5 meq/g or less, even more preferably 5.0 meq/g or less, and even more preferably 4.0 meq/g or less. The weight average molecular weight is still more preferably 20,000 or more, even more preferably 50,000 or more, and even more preferably 100,000 or more, and is still more preferably 1,000,000 or less, even more preferably 600,000 or less, even more preferably 500,000 or less, and even more preferably 300,000 or less.

The cationic charge density of the acrylic acid-methyl acrylate-methacrylamidopropyltrimethylammonium chloride copolymer (polyquaternium-47) is even more preferably 2.0 meq/g or more, and even more preferably 2.5 meq/g or more, and is even more preferably 6.5 meq/g or less, even more preferably 5.0 meq/g or less, and even more preferably 4.5 meq/g or less. The weight average molecular weight is still more preferably 200,000 or more, even more preferably 500,000 or more, and even more preferably 1,000,000 or more, and is preferably 5,000,000 or less, more preferably 3,000,000 or less, and still more preferably 1,500,000 or less.

The cationic charge density of the vinylimidazolium trichloride-vinylpyrrolidone copolymer (polyquaternium-16) is even more preferably 2.0 meq/g or more, and is even more preferably 6.5 meq/g or less, even more preferably 5.0 meq/g or less, and even even more preferably 4.5 meq/g or less. The weight average molecular weight is still more preferably 20,000 or more, and even more preferably 50,000 or more, and is still more preferably 150,000 or less.

Commercially available polymers can also be used as the component (A). Specific examples thereof include "MERQUAT 2001 Polymer" (acrylic acid-methyl acrylate-methacrylamidopropyltrimethylammonium chloride copolymer; polyquaternium-47, cationic charge density: 3.21 meq/g, Mw: 1,300,000), "MERQUAT 100" (dimethyldiallylammonium chloride polymer; polyquaternium-6, cationic charge density: 6.18 meq/g, Mw: 150,000), "MERQUAT 106" (dimethyldiallylammonium chloride polymer; polyquaternium-6, cationic charge density: 6.18 meq/g, Mw: 150,000), "MERQUAT 3940 POLYMER" (acrylamide-acrylic acid-dimethyl diallyl ammonium chloride polymer liquid; polyquaternium-39, cationic charge density: 2.97 meq/g, Mw: 150,000), "MERQUAT 280" (dimethyldiallylammonium chloride-acrylic acid copolymer liquid; polyquaternium-22, cationic charge density: 4.99 meq/g, Mw: 450,000), "MERQUAT 295" (dimethyldiallylammonium chloride-acrylic acid copolymer liquid; polyquaternium-22, cationic charge density: 6.04 meq/g, Mw: 190,000), "MERQUAT 2003PR Polymer" (acrylic acid-acrylamide-methacrylamidopropyltrimethyl ammonium chloride copolymer; polyquaternium-53), "MERQUAT 550" (diallyldimethylammonium chloride-acrylamide copolymer; polyquaternium-7), "MERQUAT 740" (diallyldimethylammonium chloride-acrylamide copolymer; polyquaternium-7, cationic charge density: 2.59 meq/g, Mw: 120,000) manufactured by Lubrizol Advanced Materials, Inc., "Luviquat FC 550" (vinylimidazolium trichloride-vinylpyrrolidone copolymer; polyquaternium-16, cationic charge density: 3.91 meq/g, Mw: 80,000), "Luviquat Excellence" (vinylimidazolium trichloride-vinylpyrrolidone copolymer; polyquaternium-16, cationic charge density: 6.65 meq/g, Mw: 40,000), "Luviquat FC 370" (vinylimidazolium trichloride-vinylpyrrolidone copolymer; polyquaternium-16, cationic charge density: 2.48 meq/g, Mw: 100,000) manufactured by BASF SE, "POIZ C-60H" manufactured by Kao Corporation (vinylimidazolium trichloride-vinylpyrrolidone copolymer; polyquaternium-16, cationic charge density: 1.5 meq/g, Mw: 600,000), "KP Polymer E" manufactured by Kao Corporation (methacryloyl ethyl trimethyl ammonium chloride copolymer (polyquaternium-37), cationic charge density: 4.8 meq/g, Mw: 300,000), "Plascize L-440" and "Plascize L-440W" (methacryloyl ethyl dimethyl betaine-methacryloyl ethyl trimethyl ammonium chloride-methoxypolyethylene glycol methacrylate copolymer; polyquaternium-49), and "Plascize L-450" and "Plascize L-450W" (methacryloyl ethyl dimethyl betaine-methacryloyl ethyl trimethyl ammonium chloride-2-hydroxyethyl methacrylate copolymer; polyquaternium-48) manufactured by Goo Chemical Co., Ltd..

### (Component (B): Anionic Polymer)

The component (B) is a polymer capable of forming a polyion complex by interaction with the component (A). The term "anionic polymer" in the description herein means a polymer having an anionic group and substantially having no cationic group and amphoteric group. The phrase "substantially having no cationic group and amphoteric group" means that the molar amount of the cationic group and the amphoteric group with respect to the anionic group is preferably 0.1% or less.

The anionic group of the component (B) is preferably an acidic group such as a carboxy group, a sulfonic acid group, or a phosphoric acid group, and more preferably a carboxy group from the viewpoint of facilitating the formation of the polyion complex by interaction with the component (A) and from the viewpoint of availability. In the component (B), at least a part of the anionic group may be neutralized and may be in the form of a salt.

The anionic charge density of the component (B) is preferably 0.1 meq/g or more, more preferably 0.5 meq/g or more, still more preferably 1.0 meq/g or more, and even more preferably 2.0 meq/g or more, from the viewpoint of facilitating the formation of the polyion complex by interaction with the component (A), and is preferably 30 meq/g or less, more preferably 20 meq/g or less, and still more preferably 15 meq/g or less, from the viewpoint of stability of the emulsion composition. Thus, the anionic charge density of the component (B) is preferably 0.1 meq/g or more and 30 meq/g or less, more preferably 0.5 meq/g or more and 30 meq/g or less, still more preferably 1.0 meq/g or more and 20 meq/g or less, and even more preferably 2.0 meq/g or more and 15 meq/g or less.

The anionic charge density of the component (B) can be calculated from (the number of moles of anionic groups contained per 1 g of polymer) × 1000 (meq/g). When at least a part of the anionic groups of the component (B) is in the form of a neutralized salt, the number of moles of the anionic groups includes the number of moles of the anionic groups in the form of a salt.

The emulsion composition may use two or more polymers as the component (B), and in this case, the anionic charge density of the component (B) is obtained by calculating the weighted average from the anionic charge density and the blending amount of each polymer.

The weight average molecular weight (Mw) of the component (B) is preferably 3,000 or more, more preferably 5,000 or more, and still more preferably 10,000 or more, and is preferably 50,000 or less, more preferably 40,000 or less, and still more preferably 30,000 or less from the viewpoints of facilitating the formation of the polyion complex, stability of the emulsion composition, and improvement in feel at the time of application to the hair and suppression of spread of the hair under high humidity conditions when the emulsion composition is used as a hair cosmetic composition. Thus, the weight average molecular weight (Mw) of the component (B) is preferably 3,000 or more and 50,000 or less, more preferably 5,000 or more and 40,000 or less, and still more preferably 10,000 or more and 30,000 or less. The weight average molecular weight of the component (B) can be measured by gel permeation chromatography (GPC).

Examples of the polymer having a carboxy group used as the component (B) include a carboxy group-containing cellulose derivative, a carboxyvinyl polymer, poly(meth)acrylic acid, a (meth)acrylic acid-based copolymer, and salts thereof. Here, the term "(meth)acrylic acid" means acrylic acid or methacrylic acid.

Among the above, examples of the carboxy group-containing cellulose derivative include carboxymethyl cellulose, carboxyethyl cellulose, carboxymethylethyl cellulose, and salts thereof.

Examples of the (meth)acrylic acid-based copolymer include a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/2-hydroxyethyl methacrylate copolymer, a (meth)acrylic acid/alkyl (meth)acrylate copolymer, an acrylic acid/alkyl acrylate/(N-alkyl)acrylamide copolymer, a (meth)acrylic acid/alkyl (meth)acrylate copolymer, an (acrylic acid/diacetone acrylamide) copolymer AMP, an (acrylic acid/alkyl acrylate/diacetone acrylamide) copolymer AMP, a (meth)acrylic acid/alkyl acrylate/polyvinylpyrrolidone copolymer, a 2- ethylhexyl acrylate/methyl acrylate/acrylic acid/methacrylic acid glycidyl copolymer, and salts thereof.

Examples of the (meth)acrylic acid/alkyl (meth)acrylate copolymer include a copolymer of (meth)acrylic acid and alkyl (meth)acrylate having an alkyl group having 1 or more and 24 or less, preferably 8 or more and 22 or less carbon atoms, such as an (acrylic acid/octyl acrylate) copolymer and an (acrylic acid/stearyl acrylate) copolymer.

The component (B) can be used alone or in combination of two or more thereof. Among the above, from the viewpoints of facilitating the formation of the polyion complex, stability of the emulsion composition, improvement in feel at the time of application to the hair, and suppression of spread of the hair under high humidity conditions, the component (B) is preferably one or more selected from the group consisting of poly(meth)acrylic acid, a (meth)acrylic acid-based copolymer, and salts thereof, more preferably one or more selected from the group consisting of a polyacrylic acid, a (meth)acrylic acid/alkyl (meth)acrylate copolymer, and salts thereof, and still more preferably one or more selected from the group consisting of polyacrylic acid, an (acrylic acid/stearyl acrylate) copolymer, and salts thereof.

Commercially available polymers can also be used as the component (B). Specific examples thereof include "Carbopol 980" and "Carbopol 981" (carboxyvinyl polymer) manufactured by Lubrizol Advanced Materials, Inc., "ACUSOL 445G" (sodium polyacrylate) manufactured by The Dow Chemical Company, "SOFCARE SA-37W" ((acrylic acid/stearyl acrylate) copolymer) manufactured by Kao Corporation, "Diahold" ((meth)acrylic acid/alkyl (meth)acrylate copolymer) manufactured by Mitsubishi Chemical Corporation, "Ultrahold 8", "Ultrahold Strong", and "Ultrahold Power" manufactured by BASF Japan Ltd., "UNFOAMER V-42" (acrylic acid/alkyl acrylate/(N-alkyl)acrylamide copolymer) manufactured by National Starch & Chemical Company, "Plascize L-53P", "Plascize L-75CB", "Plascize L-9540B", "Plascize L-6466", and "Plascize L-3200B" ((acrylic acid/diacetone acrylamide) copolymer AMP or (acrylic acid/alkyl acrylate/diacetone acrylamide) copolymer AMP) manufactured by Goo Chemical Co., Ltd., and "Luviflex VBM35" ((meth)acrylic acid/alkyl acrylate/polyvinylpyrrolidone copolymer) manufactured by BASF SE.

### (Component (C): Organic Acid)

The component (C) contributes to stably forming a polyion complex in the mixture A in the step (1). It is considered that the ionic interaction between the component (A) and the component (B) is weakened by the component (C), and thus the polyion complex is dispersed without aggregation in water.

Examples of the organic acid as the component (C) include carboxylic acid-based compounds and sulfonic acid-based compounds other than the component (B). The component (C) is preferably a compound having a molecular weight of 500 or less, and more preferably 200 or less, from the viewpoint of distinguishing from the component (B).

Examples of the carboxylic acid-based compound used as the component (C) include aliphatic monocarboxylic acids having 4 or less carbon atoms such as acetic acid, propionic acid, and butanoic acid; aromatic monocarboxylic acids such as benzoic acid; aliphatic dicarboxylic acids such as malonic acid, succinic acid, glutaric acid, adipic acid, maleic acid, and fumaric acid; aromatic dicarboxylic acids such as phthalic acid and isophthalic acid; polycarboxylic acids such as polyglutamic acid; hydroxycarboxylic acids such as lactic acid, malic acid, glycolic acid, hydroxyacrylic acid, glyceric acid, tartaric acid, and citric acid; and acidic amino acids such as glutamic acid and aspartic acid; and One, or two or more of these can be used.

Examples of the sulfonic acid-based compound used as the component (C) include aliphatic sulfonic acids such as methanesulfonic acid and ethanesulfonic acid; and aromatic sulfonic acids such as p-toluenesulfonic acid and naphthalenesulfonic acid, and one or more of these can be used.

From the viewpoint of stably forming a polyion complex in the step (1) and forming an emulsion composition having high stability through the step (2), and from the viewpoint of using in a cosmetic composition, the component (C) is preferably one or more selected from the group consisting of aliphatic dicarboxylic acids, hydroxycarboxylic acids, and aromatic sulfonic acids, more preferably one or more selected from the group consisting of succinic acid, lactic acid, malic acid, glycolic acid, p-toluenesulfonic acid, and naphthalenesulfonic acid, still more preferably one or more selected from the group consisting of succinic acid and lactic acid, and even more preferably lactic acid.

At least a part of the component (C) may be in the form of an organic acid salt when the component (C) is blended. As the salt of the organic acid, an alkali metal salt or an alkali metal salt of the organic acid is preferable, an alkali metal salt is more preferable, and one or more selected from the group consisting of a sodium salt and a potassium salt is still more preferable, and a sodium salt is even more preferable, from the viewpoint of using in a cosmetic composition or the like, and from the viewpoint of easiness of availability.

The proportion of the organic acid salt in the component (C) is preferably 70% by mass or less, more preferably 50% by mass or less, still more preferably 35% by mass or less, even more preferably 20% by mass or less, even more preferably 10% by mass or less, even more preferably 5% by mass or less, and even more preferably 2% by mass or less, and may be 0% by mass, from the viewpoints of facilitating the formation of the polyion complex, stability of the emulsion composition, and improvement in feel at the time of application to the hair and suppression of spread of hair under high humidity conditions when used as a hair cosmetic composition,. The proportion (% by mass) of the organic acid salt in the component (C) herein means % by mass converted to an organic acid.

### (Component (D): Water)

The component (D) is preferably deionized water or distilled water. Tap water, ground water, or the like sterilized with hypochlorous acid or the like may be used within a range that does not impair the stability of the emulsion composition.

In the emulsion composition, it is preferable to blend an ionic surfactant and an amphiphilic substance having a hydrophobic group as emulsion forming components in addition to the components (A) to (D) used in the step (1). Particularly, when the emulsion composition is used as a hair cosmetic composition, particularly as a hair rinse, hair conditioner, hair treatment, or hair styling agent, from the viewpoint of further improving the feel at the time of application to the hair and the feel of the hair after treatment, the ionic surfactant is preferably a cationic surfactant, and the amphiphilic substance having a hydrophobic group is preferably a higher alcohol. That is, the emulsion composition contains, as emulsion forming components, preferably component (E): a cationic surfactant and component (F): a higher alcohol. Furthermore, as optional components, component (G): an oil agent and component (H): an aqueous medium can be blended in the emulsion composition.

### (Component (E): Cationic Surfactant)

The cationic surfactant as the component (E) is an emulsion forming component contained in the oil phase of the emulsion composition.

Examples of the cationic surfactant include (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and a salt thereof, (vi) an alkoxyalkyldimethylamine and a salt thereof, and (vii) an alkylamidoalkyldimethylamine and a salt thereof.

The alkyltrimethylammonium salt (i) includes an alkyltrimethylammonium salt having an alkyl group having preferably 12 or more and 22 or less carbon atoms, and more preferably 16 or more and 20 or less carbon atoms, and specific examples thereof include cetyltrimethylammonium chloride (cetrimonium chloride), stearyltrimethylammonium chloride (steartrimonium chloride), and behenyltrimethylammonium chloride (behentrimonium chloride).

The alkoxyalkyltrimethylammonium salt (ii) includes an alkoxyalkyltrimethylammonium salt having an alkoxy group having preferably 12 or more and 22 or less carbon atoms, and more preferably 16 or more and 20 or less carbon atoms, and specific examples thereof include stearoxypropyltrimethylammonium chloride, stearoxyethyltrimethylammonium chloride, and stearoxyhydroxypropyltrimethylammonium chloride.

The dialkyldimethylammonium salt (iii) includes a dialkyldimethylammonium salt having an alkyl group having preferably 12 or more and 22 or less carbon atoms, and more preferably 16 or more and 20 or less carbon atoms, and specific examples thereof include distearyldimethylammonium chloride.

The alkylamidoalkyltrimethylammonium salt (iv) includes an alkylamidoalkyltrimethylammonium salt in which the alkyl carbon number of the alkylamido moiety is preferably 11 or more and 21 or less, and more preferably 13 or more and 19 or less, and specific examples thereof include palmitamidopropyltrimethylammonium chloride (palmitamidopropyltrimonium chloride).

The alkyldimethylamine (v), the alkoxyalkyldimethylamine (vi), and the alkylamidoalkyldimethylamine (vii) each react with an acid to become a tertiary amine salt, and become a cationic surfactant.

The alkyl group in the alkyldimethylamine and a salt thereof (v), and the alkoxy group in the alkoxyalkyldimethylamine and a salt thereof (vi) each has preferably 12 or more and 22 or less carbon atoms, and more preferably 16 or more and 20 or less carbon atoms.

The alkyl carbon number of the alkylamide moiety in the alkylamidoalkyldimethylamine and a salt thereof (vii) is preferably 11 or more and 21 or less, and more preferably 15 or more and 19 or less.

The amines (v) to (vii) may be reacted with an acid in advance and then blended into the emulsion composition as a salt, or may be blended into the emulsion composition as an amine and then an acid may be blended into the emulsion composition to form a salt in the composition. Accordingly, the amines and their salts are defined herein as cationic surfactants. The content or the blending amount of the amine is converted in terms of the mass of the amine

Examples of the salt of the amines (v) to (vii) include salts with an organic acid or an inorganic acid. Examples of the organic acid include monocarboxylic acids such as acetic acid and propionic acid; dicarboxylic acids such as malonic acid, succinic acid, glutaric acid, adipic acid, maleic acid, fumaric acid, and phthalic acid; polycarboxylic acids such as polyglutamic acid; hydroxycarboxylic acids such as glycolic acid, lactic acid, hydroxyacrylic acid, glyceric acid, malic acid, tartaric acid, and citric acid; and acidic amino acids such as glutamic acid and aspartic acid. Examples of the inorganic acid include hydrochloric acid, sulfuric acid, and phosphoric acid. Among these, organic acids are preferable, and one or more selected from the group consisting of dicarboxylic acids, hydroxycarboxylic acids, and acidic amino acids are more preferable. The dicarboxylic acid is more preferably one or more selected from the group consisting of maleic acid and succinic acid. The hydroxycarboxylic acid is more preferably one or more selected from the group consisting of glycolic acid, lactic acid, and malic acid. As the acidic amino acid, glutamic acid is more preferable.

Examples of the alkyldimethylamine and a salt thereof (v) include N,N-dimethylbehenylamine, N,N-dimethylstearylamine, and organic acid salts thereof, and a lactic acid salt of N,N-dimethylbehenylamine and a glycolic acid salt of N,N-dimethylstearylamine are preferable.

Examples of the alkoxyalkyldimethylamine and a salt thereof (vi) include N,N-dimethyl-3-hexadecyloxypropylamine, N,N-dimethyl-3-octadecyloxypropylamine (stearoxypropyldimethylamine), and organic acid salts thereof, and N,N-dimethyl-3-hexadecyloxypropylamine or a lactic acid salt thereof, and N,N-dimethyl-3-octadecyloxypropylamine (stearoxypropyldimethylamine) or a lactic acid salt thereof are preferable.

Examples of the alkylamidoalkyldimethylamine and a salt thereof (vii) include N-[3-(dimethylamino)propyl]docosanamide and N-[3-(dimethylamino)propyl]stearamide, and organic acid salts thereof, and among these, a lactic acid salt of N-[3-(dimethylamino)propyl]docosanamide and a glycolic acid salt of N-[3-(dimethylamino)propyl]stearamide are preferable.

One, or two or more of the component (E) can be used.

Among the above, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent, the component (E) is preferably one or more selected from the group consisting of the alkyltrimethylammonium salt (i), the alkoxyalkyltrimethylammonium salt (ii), the dialkyldimethylammonium salt (iii), the alkylamidoalkyltrimethylammonium salt (iv), the alkyldimethylamine and a salt thereof (v), the alkoxyalkyldimethylamine and a salt thereof (vi), and the alkylamidoalkyldimethylamine and a salt thereof (vii), more preferably one or more selected from the group consisting of the alkoxyalkyltrimethylammonium (ii) and the alkyldimethylamine and a salt thereof (v), still more preferably one or more selected from the group consisting of stearoxypropyltrimethylammonium chloride, stearoxyethyltrimethylammonium chloride, stearoxyhydroxypropyltrimethylammonium chloride, N,N-dimethyl-3-hexadecyloxypropylamine or a lactic acid salt thereof, and N,N-dimethyl-3-octadecyloxypropylamine (stearoxypropyldimethylamine) or a lactic acid salt thereof, and even more preferably one or more selected from the group consisting of stearoxypropyltrimethylammonium chloride and N,N-dimethyl-3-octadecyloxypropylamine (stearoxypropyldimethylamine) or a lactic acid salt thereof.

### (Component (F): Higher Alcohol)

The higher alcohol as the component (F) is an emulsion forming component contained in the oil phase of the emulsion composition.

Examples of the component (F) include aliphatic monohydric alcohols having 12 or more carbon atoms, preferably 12 or more and 22 or less carbon atoms. The higher alcohol may be one or more selected from the group consisting of lauryl alcohol, cetyl alcohol, myristyl alcohol, oleyl alcohol, stearyl alcohol, isostearyl alcohol, 2-octyldodecanol, behenyl alcohol, and cetostearyl alcohol.

Among the above, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent, one or more selected from the group consisting of cetyl alcohol, myristyl alcohol, stearyl alcohol, and behenyl alcohol are preferable, and stearyl alcohol is more preferable.

### (Component (G): Oil Agent)

The oil agent as the component (G) is a component contained in the oil phase of the emulsion composition other than the component (F). The component (G) may be blended in the oil phase component containing the emulsion forming component, or may be blended as another oil phase component.

Examples of the component (G) include (a) silicone oil, (b) ester oil, (c) ether oil, (d) hydrocarbon oil, (e) higher fatty acid, and (f) wax, and One, or two or more of these can be used.

As the silicone oil (a), one or more selected from the group consisting of dimethylpolysiloxane (dimethicone), dimethiconol (dimethylpolysiloxane having a hydroxy group at the terminal), methylphenylpolysiloxane, and modified silicone are preferable.

Examples of the modified silicone include amino-modified silicone (dimethylpolysiloxane having an amino group, such as amodimethicone), polyether-modified silicone, aminopolyether-modified silicone (silicone having an amino group and a polyether structure), glyceryl-modified silicone, carboxy-modified silicone, fatty acid-modified silicone, alcohol-modified silicone, aliphatic alcohol-modified silicone, epoxy-modified silicone, fluorine-modified silicone, and alkyl-modified silicone.

Among the above, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent, the silicone oil (a) is preferably one or more selected from the group consisting of dimethylpolysiloxane, dimethiconol, methylphenylpolysiloxane, and modified silicone, more preferably one or more selected from the group consisting of dimethylpolysiloxane, dimethiconol, amino-modified silicone, and polyether-modified silicone, and still more preferably one or more selected from the group consisting of dimethylpolysiloxane, amino-modified silicone, and aminopolyether-modified silicone.

Examples of the ester oil (b) include synthetic ester oils and natural oils and fats, and examples thereof include esters of a monovalent carboxylic acid and a monohydric alcohol, esters of a monovalent carboxylic acid and a polyhydric alcohol, and esters of a polyvalent carboxylic acid and a monohydric alcohol.

Examples of the ester of a monovalent carboxylic acid and a monovalent alcohol include an ester represented by the following general formula (1).

R¹-COO-R² (1)

In the general formula (1), R¹ represents a linear or branched alkyl group or alkenyl group having 1 or more and 25 or less carbon atoms, or an aromatic-containing hydrocarbon group having 6 or more and 24 or less carbon atoms, which may be substituted with a hydroxy group, and R² represents a linear or branched alkyl group or alkenyl group having 1 or more and 30 or less carbon atoms.

When R¹ is an alkyl group or an alkenyl group, the number of carbon atoms is preferably 7 or more, and is preferably 23 or less, more preferably 21 or less, still more preferably 19 or less, and even more preferably 17 or less.

When R¹ is an aromatic-containing hydrocarbon group, the number of carbon atoms is preferably 6 or more, and is preferably 22 or less, and more preferably 20 or less.

The number of carbon atoms of R² is preferably 2 or more, and is preferably 28 or less, more preferably 24 or less, and still more preferably 20 or less.

Even more preferably, in the ester represented by the general formula (1), R¹ is a linear or branched alkyl group having 7 or more and 17 or less carbon atoms, and R² is a linear or branched alkyl group having 2 or more and 24 or less carbon atoms.

Specific examples of the ester represented by the general formula (1) include one or more selected from the group consisting of cetyl isooctanoate, stearyl isooctanoate, isononyl isononanoate, isotridecyl isononanoate, hexyl laurate, isostearyl laurate, butyl myristate, isopropyl myristate, decyl myristate, isotridecyl myristate, isocetyl myristate, isostearyl myristate, 2-octyldodecyl myristate, isopropyl palmitate, 2-ethylhexyl palmitate, isostearyl palmitate, 2-hexyldecyl palmitate, 2-ethylhexyl stearate, 2-hexyldecyl stearate, isopropyl isostearate, 2-hexyldecyl isostearate, ethyl oleate, isodecyl oleate, oleyl oleate, octyldodecyl oleate, ethyl linoleate, isopropyl linoleate, lanolin acetate, methyl castor oil fatty acid (methyl ricinoleate), and alkyl benzoate (alkyl having 12 to 15 carbon atoms).

Examples of the ester of a monovalent carboxylic acid and a monovalent alcohol also include an ester represented by the following general formula (2).

R³-COO-(AO)ₙ-R⁴ (2)

In the general formula (2), R³ represents a linear or branched alkyl group or alkenyl group having 1 or more and 25 or less carbon atoms, which may be substituted with a hydroxy group, and R⁴ represents an aromatic-containing hydrocarbon group having 6 or more and 24 or less carbon atoms. AO represents an alkyleneoxy group having 2 or more and 4 or less carbon atoms, and n represents an average addition mole number of 1 or more and 50 or less.

R³ is preferably an alkyl group having 7 or more carbon atoms, and having preferably 23 or less, more preferably 21 or less, and still more preferably 19 or less carbon atoms.

R⁴ is preferably an aromatic-containing hydrocarbon group having 6 or more carbon atoms, and having preferably 22 or less, more preferably 20 or less, and still more preferably 18 or less carbon atoms, and even more preferably a benzyl group.

The AO group is preferably a propyleneoxy group, and n is preferably 1 or more and 10 or less, and more preferably 1 or more and 5 or less.

Specific examples of the ester represented by the general formula (2) include an ester of myristic acid with 3 mol propylene oxide adduct of benzyl alcohol ("Crodamol STS" manufactured by Croda International PLC), and an ester of 2-ethylhexanoic acid with 3 mol propylene oxide adduct of benzyl alcohol ("Crodamol SFX" manufactured by Croda International PLC).

Examples of the ester of a monovalent carboxylic acid and a monovalent alcohol include an ester represented by the following general formula (3).

R⁵-(OCOR⁶)ₚ (3)

In the general formula (3), R⁵ represents a polyhydric alcohol residue, and preferably a hydrocarbon group having 2 or more and 10 or less carbon atoms, R⁶ represents a monovalent carboxylic acid residue having 1 or more and 25 or less carbon atoms, and p represents an integer of 2 or more and 10 or less.

R⁵ may have an ether bond, but is preferably a linear or branched hydrocarbon group having 2 or more and 10 or less carbon atoms. The number of p is the same as the number of hydroxy groups of the polyhydric alcohol.

R⁶ is preferably an alkyl group having 7 or more carbon atoms, and from the same viewpoint as described above, is preferably an alkyl group having 23 or less carbon atoms, more preferably 21 or less carbon atoms, and still more preferably 19 or less carbon atoms.

Even more preferably, in the ester represented by the general formula (3), R⁵ is a glycerin residue or a pentaerythritol residue, and R⁶ is an alkyl group having 7 or more and 19 or less carbon atoms.

Specific examples of the ester represented by the general formula (3) include propylene glycol dicaprate, propylene glycol dioleate, neopentyl glycol dicaprate, neopentyl glycol di-2-ethylhexanoate, propanediol di(capryate/caprate), propanediol diisostearate, ethylene glycol di-2-ethylhexanoate, glyceryl tri(caprylate/caprate), glyceryl tri-2-ethylhexylate, glyceryl tri-2-heptylundecanoate, trimethylolpropane tri-2-ethylhexylate, trimethylolpropane triisostearate, pentaerythritol tetra2-ethylhexylate, and natural fats and oils.

Examples of the natural fats and oils include triglycerides such as avocado oil, olive oil, sesame oil, rice bran oil, safflower oil, soybean oil, corn oil, rapeseed oil, castor oil, cottonseed oil, and mink oil.

Examples of the ester of a polyvalent carboxylic acid and a monovalent alcohol also include an ester represented by the following general formula (4).

R⁷-(COOR⁸)_{q} (4)

In the general formula (4), R⁷ is a polyvalent carboxylic acid residue having 2 or more and 10 or less carbon atoms, R⁸ represents a monovalent alcohol residue having 1 or more and 24 or less carbon atoms, and q is an integer of 2 or more and 10 or less. Further, the number of q is the same as the number of carboxy groups of the polyvalent carboxylic acid.

R⁸ preferably has 3 or more carbon atoms, more preferably 6 or more carbon atoms, and from the same viewpoint as described above, preferably has 22 or less, more preferably 20 or less, and still more preferably 18 or less carbon atoms.

Even more preferably, in ester represented by the general formula (4), R⁷ is a trimellitic acid residue and R⁸ is a branched alkyl group having 6 or more and 18 or less carbon atoms.

Specific examples of the ester represented by the general formula (4) include diisostearyl malate, di-2-ethylhexyl succinate, diisobutyl adipate, di-2-heptylundecyl adipate, di-2-ethylhexyl sebacate, diisopropyl sebacate, and tri-2-ethylhexyl trimellitate.

Among these, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent, esters of a monovalent carboxylic acid and a monohydric alcohol represented by the general formula (1), esters of a monovalent carboxylic acid and a polyhydric alcohol represented by the general formula (3), or esters of a polyvalent carboxylic acid and a monohydric alcohol represented by the general formula (4), is preferable, and one or more selected from the group consisting of esters of the general formula (1) in which R¹ is a linear or branched alkyl group having 7 or more and 17 or less carbon atoms, and R² is a linear or branched alkyl group having 2 or more and 24 or less carbon atoms, esters of the general formula (3) in which R⁵ is a glycerin residue and R⁶ is an alkyl group having 7 or more and 19 or less carbon atoms, and esters of the general formula (4) in which R⁷ is a trimellitic acid residue and R⁸ is a branched alkyl group having 6 or more and 18 or less carbon atoms, are more preferable.

Examples of the ether oil (c) include a dialkyl ether compound represented by the following general formula (5) or a polyoxyalkylene alkyl ether compound represented by the following general formula (6).

R⁹-O-R¹⁰ (5)

In the general formula (5), R⁹ and R¹⁰ each independently represent a linear or branched alkyl group or alkenyl group having 6 or more and 22 or less carbon atoms, or an aromatic hydrocarbon group having 6 or more and 24 or less carbon atoms.

R⁹ and R¹⁰ are each preferably an alkyl group, and the number of carbon atoms thereof is preferably 8 or more, and is preferably 18 or less, more preferably 16 or less, and still preferably 12 or less, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent.

R¹¹-O-(PO)r(EO)s-H (6)

In the general formula (6), R¹¹ represents an alkyl group or alkenyl group having 6 or more and 22 or less carbon atoms, PO represents a propyleneoxy group, and EO represents an ethyleneoxy group. r represents an average addition mole number of 0.1 or more and 15 or less, and s represents an average addition mole number of 0 or more and 10 or less. When s is not 0, the addition form of PO and EO may be random or block.

The number of carbon atoms of R¹¹ is preferably 8 or more, and is preferably 20 or less, more preferably 18 or less, and still more preferably 12 or less.

The average addition mole number r is preferably 1 or more, more preferably 2 or more, and still more preferably 3 or more, and is preferably 13 or less, and more preferably 10 or less, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent, and the average addition mole number s is preferably 5 or less, more preferably 1 or less, and still more preferably 0.

The polyoxyalkylene alkyl ether compound represented by the general formula (6) is preferably one or more selected from the group consisting of polypropylene glycol, polyoxypropylene octyl ether, polyoxypropylene decyl ether, and polyoxypropylene lauryl ether in which the average addition mole number r of the propyleneoxy group is 3 or more and 10 or less.

Examples of the hydrocarbon oil (d) include squalene, squalane, liquid paraffin, liquid isoparaffin, isohexadecane, isoeicosane, hydrogenated polyisobutene, light liquid isoparaffin, heavy liquid isoparaffin, α-olefin oligomer, and cycloparaffin.

Examples of the higher fatty acid (e) include fatty acids having 8 or more carbon atoms, preferably 12 or more carbon atoms, and examples thereof include capric acid, lauric acid, myristic acid, palmitic acid, stearic acid, behenic acid, oleic acid, coconut oil fatty acid, lanolin fatty acid, isostearic acid, and isopalmitic acid.

Examples of the wax (f) include beeswax, whale wax, lanolin, and carnauba wax.

As the oil agent as the component (G), one or more of the above can be used. Among the above, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent, the component (G) preferably contains one or more selected from the group consisting of (a) silicone oil, (b) ester oil, (d) hydrocarbon oil, and (e) higher fatty acid, more preferably contains one or more selected from the group consisting of (a) silicone oil, and (e) higher fatty acid, still more preferably contains one or more selected from the group consisting of dimethylpolysiloxane, dimethiconol, methylphenylpolysiloxane, modified silicone, and fatty acids having 12 or more carbon atoms, even more preferably contains one or more selected from the group consisting of dimethylpolysiloxane, dimethiconol, amino-modified silicone, polyether-modified silicone, aminopolyether-modified silicone, lauric acid, myristic acid, palmitic acid, stearic acid, behenic acid, oleic acid, coconut oil fatty acid, lanolin fatty acid, isostearyl acid, and isopalmitic acid, even more preferably contains one or more selected from the group consisting of dimethylpolysiloxane, amino-modified silicone, aminopolyether-modified silicone, and lanolin fatty acid, and even more preferably contains one or more selected from the group consisting of dimethylpolysiloxane, amino-modified silicone, and lanolin fatty acid.

### (Component (H): Aqueous Medium)

The aqueous medium as the component (H) means an aqueous medium other than water. The component (H) is a component contained in the aqueous phase of the emulsion composition, but may be blended in the oil phase component containing the emulsion forming component.

Examples of the component (H) include lower alcohols such as ethanol and isopropyl alcohol; and low molecular weight diols and triols having 6 or less carbon atoms, such as 1,3-butylene glycol, glycerin, ethylene glycol, propylene glycol, and dipropylene glycol. One, or two or more of these can be used.

### (Blending Amount)

The blending amounts or contents of the respective components in the emulsion composition are preferably as follows from the viewpoints of stability of the emulsion composition, improvement in feel at the time of application to the hair, and suppression of spread of the hair under high humidity conditions.

The blending amount of the component (A) in the emulsion composition is an amount of preferably 0.01% by mass or more, more preferably 0.02% by mass or more, and still more preferably 0.03% by mass or more, based on 100% by mass of the total amount of the emulsion composition, from the viewpoint of facilitating the formation of the polyion complex, from the viewpoint of improving the feel at the time of application to the hair when the obtained emulsion composition is used as a hair cosmetic composition, and from the viewpoint of suppressing the spread of the hair under high humidity conditions. On the other hand, from the viewpoints of improving the emulsified state and improving the application feel when the obtained emulsion composition is used as a hair cosmetic composition and applied to the hair, the blending amount is preferably less than 0.50% by mass, more preferably 0.30% by mass or less, and still more preferably 0.20% by mass or less. Thus, the blending amount of the component (A) in the emulsion composition is an amount of preferably 0.01% by mass or more and less than 0.50% by mass, more preferably 0.02% by mass or more and 0.30% by mass or less, and still more preferably 0.03% by mass or more and 0.20% by mass or less, based on 100% by mass of the total amount of the emulsion composition.

The blending amount of the component (B) in the emulsion composition is an amount of preferably 0.010% by mass or more, and more preferably 0.015% by mass or more, based on 100% by mass of the total amount of the emulsion composition, from the viewpoint of facilitating the formation of the polyion complex, from the viewpoint of improving the feel at the time of application to the hair when the obtained emulsion composition is used as a hair cosmetic composition, and from the viewpoint of suppressing the spread of the hair under high humidity conditions. On the other hand, from the viewpoint of improving the emulsified state and improving the application feel when the obtained emulsion composition is used as a hair cosmetic composition and applied to the hair, the blending amount is preferably less than 0.50% by mass, more preferably 0.30% by mass or less, still more preferably 0.20% by mass or less, even more preferably 0.15% by mass or less, and even more preferably 0.10% by mass or less. Thus, the blending amount of the component (B) in the emulsion composition is an amount of preferably 0.010% by mass or more and less than 0.50% by mass, more preferably 0.010% by mass or more and 0.30% by mass or less, still more preferably 0.015% by mass or more and 0.20% by mass or less, even more preferably 0.015% by mass or more and 0.15% by mass or less, and even more preferably 0.015% by mass or more and 0.10% by mass or less, based on 100% by mass of the total amount of the emulsion composition.

The total blending amount of the component (A) and the component (B) in the emulsion composition is less than 1.0% by mass, preferably 0.9% by mass or less, more preferably 0.5% by mass or less, and still more preferably 0.3% by mass or less, based on 100% by mass of the total amount of the emulsion composition, from the viewpoint of stability of the emulsion composition. On the other hand, from the viewpoint of suppressing the spread of the hair under high humidity conditions when the obtained emulsion composition is used as a hair cosmetic composition, the blending amount is preferably 0.01% by mass or more, more preferably 0.03% by mass or more, and still more preferably 0.05% by mass or more. Thus, the total blending amount of the component (A) and the component (B) in the emulsion composition is less than 1.0% by mass, preferably 0.01% by mass or more and 0.9% by mass or less, more preferably 0.03% by mass or more and 0.9% by mass or less, still more preferably 0.05% by mass or more and 0.9% by mass or less, even more preferably 0.05% by mass or more and 0.5% by mass or less, and even more preferably 0.05% by mass or more and 0.3% by mass or less, based on 100% by mass of the total amount of the emulsion composition.

The ratio of the number of moles of cationic charges of the component (A) to the number of moles of anionic charges of the component (B) in the emulsion composition is an amount of preferably 5/95 to 95/5, more preferably 10/90 to 90/10, still more preferably 20/80 to 80/20, even more preferably 20/80 to 60/40, and even more preferably 40/60 to 60/40, from the viewpoint of improving the emulsified state and from the viewpoint of improving the feel at the time of application to the hair when the emulsion composition is used as a hair cosmetic composition.

The blending amount of the component (C) in the emulsion composition is an amount of preferably 0.01% by mass or more, more preferably 0.02% by mass or more, still more preferably 0.03% by mass or more, even more preferably 0.05% by mass or more, even more preferably 0.1% by mass or more, even more preferably 0.2% by mass or more, and even more preferably 0.3% by mass or more, based on 100% by mass of the total amount of the emulsion composition, from the viewpoint of facilitating the formation of the polyion complex. On the other hand, from the viewpoint of facilitating the formation of the polyion complex and from the viewpoint of improving the stability of the emulsion composition obtained through the step (2-1) or (2-2), the blending amount is an amount of preferably 5.0% by mass or less, more preferably 3.0% by mass or less, still more preferably 2.0% by mass or less, even more preferably 1.5% by mass or less, and even more preferably 1.2% by mass or less. Thus, the blending amount of the component (C) in the emulsion composition is an amount of preferably 0.01% by mass or more and 5.0% by mass or less, more preferably 0.02% by mass or more and 5.0% by mass or less, still more preferably 0.03% by mass or more and 5.0% by mass or less, even more preferably 0.05% by mass or more and 3.0% by mass or less, even more preferably 0.1% by mass or more and 2.0% by mass or less, even more preferably 0.2% by mass or more and 1.5% by mass or less, and even more preferably 0.3% by mass or more and 1.2% by mass or less based on 100% by mass of the total amount of the emulsion composition.

However, when the emulsion composition contains any of the alkyldimethylamine and a salt thereof (v), the alkoxyalkyldimethylamine and a salt thereof (vi), and the alkylamidoalkyldimethylamine and a salt thereof (vii) as the component (E), the blending amount of the component (C) in the emulsion composition may be more than 5.0% by mass. This is because the component (C) is expected to improve the stability of the emulsion composition and to improve the feel at the time of application to the hair when the emulsion composition is used as a hair cosmetic composition.

When the component (C) is blended in the form of an organic acid salt, the blending amount of the component (C) is an amount converted to an organic acid. The above-mentioned blending amount of the component (C) is the total blending amount in the step (1) and the step (2-1) or (2-2).

The blending amount of the component (D) in the emulsion composition is an amount of preferably 10% by mass or more, more preferably 20% by mass or more, still more preferably 30% by mass or more, even more preferably 40% by mass or more, even more preferably 50% by mass or more, even more preferably 60% by mass or more, even more preferably 70% by mass or more, and even more preferably 80% by mass or more, based on 100% by mass of the total amount of the emulsion composition, from the viewpoint of facilitating the formation of the polyion complex and from the viewpoint of improving the emulsified state. On the other hand, from the viewpoint of the stability of the obtained emulsion composition and from the viewpoint of the degree of freedom of blending, the blending amount is an amount of preferably 99% by mass or less, more preferably 95% by mass or less, and still more preferably 90% by mass or less. Thus, the blending amount of the component (D) in the emulsion composition is an amount of preferably 10% by mass or more and 99% by mass or less, more preferably 20% by mass or more and 99% by mass or less, still more preferably 30% by mass or more and 99% by mass or less, even more preferably 40% by mass or more and 95% by mass or less, even more preferably 50% by mass or more and 95% by mass or less, even more preferably 60% by mass or more and 95% by mass or less, even more preferably 70% by mass or more and 95% by mass or less, and even more preferably 80% by mass or more and 90% by mass or less based on 100% by mass of the total amount of the emulsion composition.

The above-mentioned blending amount of the component (D) is the total blending amount in the step (1) and the step (2-1) or (2-2).

The blending amount of the cationic surfactant as the component (E) in the emulsion composition is an amount of preferably 0.1% by mass or more, more preferably 0.2% by mass or more, still more preferably 0.5% by mass or more, and even more preferably 1.0% by mass or more, based on 100% by mass of the total amount of the emulsion composition, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent. On the other hand, from the viewpoint of the stability of the obtained emulsion composition, the blending amount is an amount of preferably 10% by mass or less, more preferably 5.0% by mass or less, and still more preferably 3.5% by mass or less. Thus, the blending amount of the component (E) in the emulsion composition is an amount of preferably 0.1% by mass or more and 10% by mass or less, more preferably 0.2% by mass or more and 5.0% by mass or less, still more preferably 0.5% by mass or more and 5.0% by mass or less, and even more preferably 1.0% by mass or more and 3.5% by mass or less.

The blending amount of the higher alcohol as the component (F) in the emulsion composition is an amount of preferably 0.1% by mass or more, more preferably 0.2% by mass or more, still more preferably 0.5% by mass or more, and even more preferably 1.0% by mass or more, based on 100% by mass of the total amount of the emulsion composition, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent. On the other hand, from the viewpoint of the stability of the obtained emulsion composition, the blending amount is an amount of preferably 15% by mass or less, more preferably 12% by mass or less, and still more preferably 10% by mass or less. Thus, the blending amount of the component (F) in the emulsion composition is an amount of preferably 0.1% by mass or more and 15% by mass or less, more preferably 0.2% by mass or more and 12% by mass or less, still more preferably 0.5% by mass or more and 12% by mass or less, and even more preferably 1.0% by mass or more and 10% by mass or less.

When the oil agent is used as the component (G), the blending amount of the component (G) in the emulsion composition is an amount of preferably 0.1% by mass or more, more preferably 0.2% by mass or more, still more preferably 0.5% by mass or more, and even more preferably 1.0% by mass or more, based on 100% by mass of the total amount of the emulsion composition, from the viewpoint of further improving the feel at the time of application to the hair and the feel of the hair after treatment when the emulsion composition is used as a hair cosmetic composition, preferably a hair rinse, a hair conditioner, a hair treatment, or a hair styling agent. On the other hand, from the viewpoint of improving the feel of use of the obtained emulsion composition, the blending amount is an amount of preferably 20% by mass or less, more preferably 10% by mass or less, and still more preferably 8.0% by mass or less. Thus, the blending amount of the component (G) in the emulsion composition is an amount of preferably 0.1% by mass or more and 20% by mass or less, more preferably 0.2% by mass or more and 10% by mass or less, still more preferably 0.5% by mass or more and 10% by mass or less, and even more preferably 1.0% by mass or more and 8.0% by mass or less.

When the aqueous medium is used as the component (H), the blending amount of the component (H) in the emulsion composition is an amount of preferably 0.1% by mass or more, more preferably 0.2% by mass or more, still more preferably 0.5% by mass or more, and even more preferably 1.0% by mass or more, and is preferably 10% by mass or less, more preferably 5.0% by mass or less, and still more preferably 3.0% by mass or less, based on 100% by mass of the total amount of the emulsion composition, from the viewpoint of dispersing the emulsion forming component. Thus, the blending amount of the component (H) in the emulsion composition is an amount of preferably 0.1% by mass or more and 10% by mass or less, more preferably 0.2% by mass or more and 5.0% by mass or less, still more preferably 0.5% by mass or more and 5.0% by mass or less, and even more preferably 1.0% by mass or more and 3.0% by mass or less.

In addition, the emulsion composition may contain an anionic surfactant, a nonionic surfactant, an amphoteric surfactant, an aromatic alcohol, an antioxidant, an antidandruff agent, a vitamin agent, a bactericide, an anti-inflammatory agent, a preservative, a chelating agent, a moisturizing agent, a pearl agent, a ceramide, a perfume, a plant extract, an ultraviolet absorber, a pH modifier and the like.

### (pH)

In the emulsion composition, from the viewpoint of making the feel when the emulsion composition is applied to hair favorable, the pH of a 5% aqueous solution of the emulsion composition at 25°C is preferably 2.0 or more, more preferably 3.0 or more, and is preferably 10.0 or less, more preferably 7.0 or less, and still more preferably 5.0 or less.

The pH of a 5% aqueous solution of the emulsion composition at 25°C is a value measured by adding water to the emulsion composition to prepare a 5% aqueous solution and using a pH meter.

The method for producing the emulsion composition of the present invention is described below.

### <Step (1): Step of Preparing Mixture A>

In the step (1), the components (A) to (D) are mixed to prepare a mixture A containing the components (A) to (D). By performing the step (1), a mixture A containing a polyion complex formed from the component (A) and the component (B) is obtained.

The components (A) to (D) and details and preferred embodiments thereof are as described above.

In the preparation of the mixture A, the order of mixing the components (A) to (D) is not particularly limited, and for example, the components (A) to (C) may be simultaneously added to the component (D) and mixed. From the viewpoint of facilitating the formation of the polyion complex and from the viewpoint of forming a more homogeneous polyion complex in the component (D), it is preferable that the component (A) and the component (C) are first added to the component (D) and mixed, and then the component (B) is added and mixed.

The temperature at the time of mixing of the components (A) to (D) in the step (1) is not particularly limited, but is preferably 40°C or higher, more preferably 45°C or higher, and still more preferably 50°C or higher, from the viewpoint of improving the dispersibility of the component (A) and the component (B) and from the viewpoint of facilitating the formation of the polyion complex, and is preferably 90°C or lower, more preferably 80°C or lower, further preferably 70°C or lower, and further preferably 65°C or lower, from the viewpoint of suppressing thermal deterioration of the blending components. Thus, the temperature at the time of mixing of the components (A) to (D) in the step (1) is preferably 40°C or higher and 90°C or lower, more preferably 45°C or higher and 80°C or lower, still more preferably 50°C or higher and 70°C or lower, and even more preferably 50°C or higher and 65°C or lower.

The mixing time of the components (A) to (D) in the step (1) is not particularly limited, but is preferably 1 minute or more, and more preferably 3 minutes or more, from the viewpoint of improving the emulsified state, and is preferably 1 hour or less from the viewpoint of productivity.

The mixing of the components (A) to (D) in the step (1) can be performed using a known emulsifying machine or stirring device such as a high-pressure emulsifying machine, an ultrasonic emulsifying machine, and a homomixer.

The mixture A obtained in the step (1) may be a transparent solution, but is preferably in a suspension state. In the mixture A in a suspension state, it is considered that the polyion complex formed from the component (A) and the component (B) is not solubilized but is precipitated in the solution and uniformly dispersed. Then, it is considered that when the mixture A exhibiting a suspension state is used in the step (2-1) or (2-2), the state of the stable polyion complex is maintained in the obtained emulsion composition, and the stability of the emulsion composition, the feel at the time of application to the hair, and the effect of suppressing the spread of the hair under high humidity conditions are further improved.

In the mixture A prepared in the step (1), the preferred blending amounts of the respective components are in the following ranges.

The blending amount of the component (A) in the mixture Ais an amount of preferably 0.01% by mass or more, more preferably 0.03% by mass or more, and still more preferably 0.05% by mass or more, based on 100% by mass of the total amount of the mixture A, from the viewpoint of facilitating the formation of the polyion complex, from the viewpoint of improving the feel at the time of application to the hair when the obtained emulsion composition is used as a hair cosmetic composition, and from the viewpoint of suppressing the spread of the hair under high humidity conditions. On the other hand, from the viewpoint of improving the emulsified state, the blending amount is preferably less than 10% by mass, more preferably less than 5% by mass, still more preferably less than 1% by mass, and even more preferably less than 0.5% by mass. Thus, the blending amount of the component (A) in the mixture A is preferably 0.01% by mass or more and less than 10% by mass, more preferably 0.03% by mass or more and less than 5% by mass, still more preferably 0.03% by mass or more and less than 1% by mass, and even more preferably 0.05% by mass or more and less than 0.5% by mass based on 100% by mass of the total amount of the mixture A.

The blending amount of the component (B) in the mixture A is an amount of preferably 0.01% by mass or more, more preferably 0.02% by mass or more, and still more preferably 0.05% by mass or more, based on 100% by mass of the total amount of the mixture A, from the viewpoint of facilitating the formation of the polyion complex, from the viewpoint of improving the feel at the time of application to the hair when the obtained emulsion composition is used as a hair cosmetic composition, and from the viewpoint of suppressing the spread of the hair under high humidity conditions. On the other hand, from the viewpoint of improving the emulsified state, the blending amount is preferably less than 5% by mass, more preferably less than 3% by mass, still more preferably less than 1% by mass, and even more preferably less than 0.3% by mass. Thus, the blending amount of the component (B) in the mixture A is preferably 0.01% by mass or more and less than 5% by mass, more preferably 0.01% by mass or more and less than 3% by mass, still more preferably 0.02% by mass or more and less than 1% by mass, even more preferably 0.02% by mass or more and less than 0.3% by mass, and even more preferably 0.05% by mass or more and less than 0.3% by mass based on 100% by mass of the total amount of the mixture A.

The blending amount of the component (C) in the mixture A is an amount of preferably 0.01% by mass or more, more preferably 0.03% by mass or more, and still more preferably 0.05% by mass or more, based on 100% by mass of the total amount of the mixture A, from the viewpoint of facilitating the formation of the polyion complex. On the other hand, from the viewpoint of facilitating the preparation of the mixture A in a suspension state, the blending amount is preferably less than 10% by mass, more preferably less than 6% by mass, still more preferably less than 1% by mass, even more preferably less than 0.75% by mass, and even more preferably less than 0.30% by mass. Thus, the blending amount of the component (C) in the mixture A is preferably 0.01% by mass or more and less than 10% by mass, more preferably 0.01% by mass or more and less than 6% by mass, still more preferably 0.03% by mass or more and less than 1% by mass, even more preferably 0.05% by mass or more and less than 0.75% by mass, and even more preferably 0.05% by mass or more and less than 0.30% by mass based on 100% by mass of the total amount of the mixture A.

The amount of the component (C) used for the preparation of the mixture A in the step (1) may be a part of the component (C) contained in the obtained emulsion composition.

In the mixture A, the mass ratio of the total blending amount of the component (A) and the component (B) to the blending amount of the component (C) [(A + B)/C] is preferably in a range of 1/0.10 to 1/20, more preferably 1/0.15 to 1/15, still more preferably 1/0.20 to 1/10, even more preferably 1/0.20 to 1/8, and even more preferably 1/0.22 to 1/4, from the viewpoint of improving the feel at the time of application to the hair when the obtained emulsion composition is used as a hair cosmetic composition, and from the viewpoint of suppressing the spread of the hair under high humidity conditions.

The blending amount of the component (D) in the mixture Ais an amount of preferably 50% by mass or more, more preferably 60% by mass or more, still more preferably 70% by mass or more, and even more preferably 80% by mass or more, based on 100% by mass of the total amount of the mixture A, from the viewpoint of facilitating the formation of the polyion complex and from the viewpoint of improving the emulsified state. On the other hand, from the viewpoint of the stability of the obtained emulsion composition and from the viewpoint of the degree of freedom of blending, the blending amount is an amount of preferably 99.97% by mass or less, and more preferably 99.8% by mass or less. Thus, the blending amount of the component (D) in the mixture A is an amount of preferably 50% by mass or more and 99.97% by mass or less, more preferably 50% by mass or more and 99.8% by mass or less, still more preferably 60% by mass or more and 99.8% by mass or less, even more preferably 70% by mass or more and 99.8% by mass or less, and even more preferably 80% by mass or more and 99.8% by mass or less, based on 100% by mass of the total amount of the mixture A.

The amount of the component (D) used for the preparation of the mixture A in the step (1) may be a part of the component (D) contained in the obtained emulsion composition.

The mixture A may contain components other than the components (A) to (D), but from the viewpoint of facilitating the formation of the polyion complex, from the viewpoint of improving the emulsified state, and from the viewpoint of stability of the obtained emulsion composition, the total content of the components (A) to (D) in the mixture A is preferably 70% by mass or more, more preferably 80% by mass or more, still more preferably 90% by mass or more, and even more preferably 95% by mass or more, and is 100% by mass or less.

In the step (1), from the viewpoint of more uniformly dispersing the polyion complex in the mixture A, the pH of the mixture A at 25°C is preferably 1.5 or more, and more preferably 2.0 or more, and is preferably 4.0 or less, more preferably 3.2 or less, and still more preferably 3.0 or less. Thus, the pH of the mixture A at 25°C is preferably 1.5 or more and 4.0 or less, more preferably 2.0 or more and 4.0 or less, still more preferably 2.0 or more and 3.2 or less, and even more preferably 2.0 or more and 3.0 or less.

The pH of the mixture A at 25°C can be measured using a pH meter.

### <Step (2)>

The production method of the present invention has either of the following step (2-1) or step (2-2) after the step (1). This makes it possible to produce an emulsion composition having high stability and containing the polyion complex obtained in the step (1).
step (1): a step for preparing a mixture A containing the components (A) to (D);
step (2-1): a step for preparing an emulsion (I) by mixing the mixture A with an oil phase component containing an emulsion forming component;
step (2-2): a step for preparing an emulsion (II) by mixing an oil phase component containing an emulsion forming component with an aqueous phase component other than the mixture A, followed by mixing with the mixture A.

### (Step (2-1))

In the step (2-1), first, the mixture A obtained in the step (1) and an oil phase component containing an emulsion forming component are mixed to prepare an emulsion (I). Subsequently, one or more selected from the group consisting of an aqueous phase component other than the mixture A and an oil phase component not containing an emulsion forming component can be mixed as necessary.

The mixing ratio of the mixture A and the oil phase component containing an emulsion forming component may be adjusted and mixed so that the total blending amount of the component (A) and the component (B) in the obtained emulsion composition is within a predetermined range, and is not particularly limited.

The "oil phase component containing an emulsion forming component" preferably contains, as emulsion forming components, component (E): a cationic surfactant, and component (F): a higher alcohol, and may further contain component (H) (aqueous medium). In the oil phase component containing the emulsion forming component used in the step (2-1), the content of the emulsion forming component is preferably 30% by mass or more, more preferably 50% by mass or more, and still more preferably 70% by mass or more, and is 100% by mass or less from the viewpoint of stably forming an emulsion.

Examples of the "aqueous phase component other than the mixture A" include the remaining amount of the component (C) (organic acid) and the component (D) (water), and the component (H) (aqueous medium).

Examples of the "oil phase component not containing an emulsion forming component" include the component (G) (oil agent).

Hereinafter, the aqueous phase components other than the mixture A and the oil phase components not containing the emulsion forming components are also collectively referred to as "other components".

The temperature at the time of mixing the mixture A and the oil phase component containing the emulsion forming component in the step (2-1) is not particularly limited, but is preferably 40°C or higher, more preferably 45°C or higher, and still more preferably 50°C or higher from the viewpoint of the stability of the obtained emulsion composition, and from the viewpoint of suppressing thermal deterioration of the blending components, the temperature is preferably 90°C or lower, more preferably 80°C or lower, and still more preferably 70°C or lower. Thus, the temperature at the time of mixing the mixture A and the oil phase component containing the emulsion forming component in the step (2-1) is preferably 40°C or higher and 90°C or lower, more preferably 45°C or higher and 80°C or lower, and still more preferably 50°C or higher and 70°C or lower.

The mixing time of the mixture A and the oil phase component containing the emulsion forming component in the step (2-1) is not particularly limited, but is preferably 1 minute or more, and more preferably 3 minutes or more, from the viewpoint of improving the emulsified state, and is preferably 1 hour or less from the viewpoint of productivity.

Next, the obtained emulsion (I) and the aqueous phase components other than the mixture A and/or the oil phase components (other components) not containing the emulsion forming components are mixed as necessary.

The emulsion (I) and the other components may be mixed in one batch or may be divided into several batches, with one added to the other.

The temperature at the time of mixing of the emulsion (I) and other components is not particularly limited, but is preferably 40°C or higher, more preferably 45°C or higher, and still more preferably 50°C or higher, from the viewpoint of improving the application feel when the obtained emulsion composition is used as a hair cosmetic composition and applied to the hair, and from the viewpoint of the stability of the obtained emulsion composition, and is preferably 90°C or lower, and more preferably 80°C or lower, from the viewpoint of suppressing thermal deterioration of the blending components. When heating is performed during the preparation of the emulsion (I), it is preferable to mix the emulsion (I) with other components while performing a cooling step. The cooling step can be performed by a known method such as air cooling.

The mixing time of the emulsion (I) with other components is also not particularly limited, and is preferably 1 minute or more, 5 minutes or more, and more preferably 10 minutes or more, and from the viewpoint of productivity, it is preferably 12 hours or less, more preferably 5 hours or less, and still more preferably 1 hour or less.

### (Step (2-2))

In the step (2-2), first, an oil phase component containing an emulsion forming component and an aqueous phase component other than the mixture A are mixed to prepare an emulsion (II). Next, the emulsion and the mixture A are mixed.

The "oil phase component containing an emulsion forming component" and the "aqueous phase component other than the mixture A" are the same as in the step (2-1).

The aqueous phase component other than the mixture A used for the preparation of the emulsion (II) does not need to be the total amount of the aqueous phase components used in the step (2-2), and may be at least a part thereof. The aqueous phase component other than the mixture A used for the preparation of the emulsion (II) is preferably water of the component (D).

The temperature at the time of mixing of the oil phase component containing the emulsion forming component and the aqueous phase component other than the mixture A in the step (2-2) is not particularly limited, but is preferably 40°C or higher, more preferably 45°C or higher, and still more preferably 50°C or higher from the viewpoint of improving the application feel when the obtained emulsion composition is used as a hair cosmetic composition and applied to the hair and from the viewpoint of the stability of the obtained emulsion composition, and is preferably 90°C or lower, and more preferably 80°C or lower from the viewpoint of suppressing thermal deterioration of the blending components. Thus, the temperature at the time of mixing of the oil phase component containing the emulsion forming component and the aqueous phase component other than the mixture A in the step (2-2) is preferably 40°C or higher and 90°C or lower, more preferably 45°C or higher and 90°C or lower, and still more preferably 50°C or higher and 80°C or lower. The mixing time of the oil phase component containing the emulsion forming component and the aqueous phase component other than the mixture A in the step (2-2) is also not particularly limited, but is preferably 1 minute or more, and more preferably 3 minutes or more from the viewpoint of preparing an emulsion, and is preferably 1 hour or less from the viewpoint of productivity.

Next, the obtained emulsion (II) and the mixture A are mixed.

The mixing ratio of the mixture A and the emulsion (II) may be adjusted and mixed so that the total blending amount of the component (A) and the component (B) in the obtained emulsion composition is within a predetermined range, and is not particularly limited.

The emulsion (II) and the mixture A may be mixed in one batch or may be divided into several batches, with one added to the other. In addition, at the same time as the mixing of the emulsion (II) with the mixture A, or before or after the mixing, the remaining amount of the aqueous phase component other than the mixture A and the oil phase component (preferably the component (G) (oil agent)) not containing the emulsion forming component can be added and mixed.

The temperature at the time of mixing of the emulsion (II) and the mixture A is not particularly limited, but is preferably 50°C or lower, and more preferably 45°C or lower, and is preferably 10°C or higher, and more preferably 20°C or higher, from the viewpoint of stability of the obtained emulsion composition. Thus, the temperature at the time of mixing of the emulsion (II) and the mixture A is preferably 10°C or higher and 50°C or lower, and more preferably 20°C or higher and 45°C or lower.

The mixing time of the emulsion (II) with the mixture A is not particularly limited, and is preferably 1 minute or more, and more preferably 3 minutes or more, and from the viewpoint of productivity, the mixing time is preferably 1 hour or less.

Each mixing step in the step (2-1) and the step (2-2) can be performed using the same apparatus as that in the step (1).

The production method of the present invention may include the step (1) and any one of the steps (2-1) and (2-2). From the viewpoint of making the emulsified state more favorable, the production method of the present invention preferably includes the step (1) and the step (2-1).

The above-described emulsion composition can be obtained by the production method of the present invention. The emulsion composition has high stability and is used as a cosmetic composition, preferably a hair cosmetic composition. When the emulsion composition is used as a hair cosmetic composition, the feel at the time of application to the hair is good, and the spread hair after treatment under high humidity conditions can be suppressed.

With respect to the above-described embodiments, the present invention further discloses the following.
<1> A method for producing an emulsion composition containing the following components:
   component (A): one or more polymers selected from the group consisting of cationic polymers and amphoteric polymers;
   component (B): an anionic polymer;
   component (C): an organic acid; and
   component (D): water,
   the method including the following step (1) and the following step (2-1) or step (2-2),
   in which a total blending amount of the component (A) and the component (B) in the emulsion composition is less than 1.0% by mass based on 100% by mass of the total amount of the emulsion composition:
      step (1): a step for preparing a mixture A containing the components (A) to (D);
      step (2-1): a step for preparing an emulsion (I) by mixing the mixture A with an oil phase component containing an emulsion forming component;
      step (2-2): a step for preparing an emulsion (II) by mixing an oil phase component containing an emulsion forming component with an aqueous phase component other than the mixture A, followed by mixing with the mixture A.
<2> A method for producing an emulsion composition containing the following components:
   component (A): one or more polymers selected from the group consisting of cationic polymers and amphoteric polymers;
   component (B): an anionic polymer;
   component (C): an organic acid; and
   component (D): water,
   the method including the following step (1) and the following step (2-1) or step (2-2),
   in which a total blending amount of the component (A) and the component (B) in the emulsion composition is less than 1.0% by mass based on 100% by mass of the total amount of the emulsion composition, and the ratio of the number of moles of cationic charges of the component (A) to the number of moles of anionic charges of the component (B) in the emulsion composition is 20/80 to 60/40:
      step (1): a step of preparing a mixture A containing the components (A) to (D), in which a blending amount of the component (C) is 0.05% by mass or more and less than 0.30% by mass, and a mass ratio [(A + B)/C] of a total blending amount of the component (A) and the component (B) to a blending amount of the component (C) is 1/0.2 to 1/10;
      step (2-1): a step of mixing the mixture A and an oil phase component containing an emulsion forming component containing component (E): a cationic surfactant and component (F): a higher alcohol at a temperature of 50°C or higher and 70°C or lower to prepare an emulsion (I);
      step (2-2): a step of mixing an oil phase component containing an emulsion forming component containing component (E): a cationic surfactant and component (F): a higher alcohol, and an aqueous phase component other than the mixture A to prepare an emulsion (II), and then mixing the mixture A at a temperature of 20°C or higher and 45°C or lower.
<3> A method for producing an emulsion composition containing the following components:
   component (A): one or more polymers selected from the group consisting of a dimethyldiallylammonium chloride polymer (polyquaternium-6), a dimethyldiallylammonium chloride-acrylic acid copolymer (polyquaternium-22), an acrylamide-acrylic acid-dimethyldiallylammonium chloride copolymer (polyquaternium-39), and an acrylic acid-methyl acrylate-methacrylamidopropyltrimethylammonium chloride copolymer (polyquaternium-47);
   component (B): one or more anionic polymers selected from the group consisting of a polyacrylic acid, an (acrylic acid/stearyl acrylate) copolymer, or a salt thereof;
   component (C): an organic acid; and
   component (D): water,
   the method including the following step (1) and the following step (2-1) or step (2-2),
   in which a total blending amount of the component (A) and the component (B) in the emulsion composition is less than 1.0% by mass based on 100% by mass of the total amount of the emulsion composition, and the ratio of the number of moles of cationic charges of the component (A) to the number of moles of anionic charges of the component (B) in the emulsion composition is 20/80 to 60/40:
      step (1): a step of preparing a mixture A including the components (A) to (D), in which a blending amount of the component (C) is 0.05% by mass or more and less than 0.30% by mass, and a mass ratio [(A + B)/C] of a total blending amount of the component (A) and the component (B) to a blending amount of the component (C) is 1/0.2 to 1/10;
      step (2-1): a step of mixing the mixture A and an oil phase component containing an emulsion forming component containing component (E): a cationic surfactant and component (F): a higher alcohol at a temperature of 50°C or higher and 70°C or lower to prepare an emulsion (I);
      step (2-2): a step of mixing an oil phase component containing an emulsion forming component containing component (E): a cationic surfactant and component (F): a higher alcohol, and an aqueous phase component other than the mixture A to prepare an emulsion (II), and then mixing the mixture A at a temperature of 20°C or higher and 45°C or lower.
<4> The method for producing an emulsion composition according to <1> or <2>, in which the cationic polymer is a polymer having a cationic group and substantially having no anionic group and amphoteric group.
<5> The method for producing an emulsion composition according to any one of <1>, <2>, and <4>, in which the cationic polymer is a polymer having a cationic group and a molar amount of an anionic group and an amphoteric group with respect to the cationic group is preferably 0.1% or less.
<6> The method for producing an emulsion composition according to any one of <1>, <2>, <4>, and <5>, in which the amphoteric polymer is preferably a polymer having a cationic group and an anionic group, a pH of a 1% aqueous solution of the polymer at 25°C is less than 5.1, and the polymer is positively charged as the total charge of the polymer.
<7> The method for producing an emulsion composition according to any one of <1> to <6>, in which a cationic charge density of the component (A) is preferably 0.1 meq/g or more and 10 meq/g or less, more preferably 0.5 meq/g or more and 8.0 meq/g or less, still more preferably 1.0 meq/g or more and 7.0 meq/g or less, and even more preferably 2.0 meq/g or more and 7.0 meq/g or less.
<8> The method for producing an emulsion composition according to any one of <1> to <7>, in which a weight average molecular weight of the component (A) is preferably 5,000 or more and 2,000,000 or less and more preferably 8,000 or more and 1,500,000 or less.
<9> The method for producing an emulsion composition according to any one of <1>, <2>, and <4> to <8>, in which the component (A) is preferably one or more selected from the group consisting of a cationized guar gum, a cationized tara gum, a cationized locust bean gum, a cationic starch, a cationized cellulose, a cationized hydroxyalkyl cellulose, a cationized polyvinyl alcohol, a polyethyleneimine, a quaternized dialkylaminoalkyl (meth)acrylate polymer, a diallyl quaternized ammonium salt polymer, a methacrylamidopropyltrimethylammonium salt polymer, a methacryloyl ethyltrimethylammonium salt polymer, a vinyl imidazolium trichloride-vinyl pyrrolidone copolymer (polyquaternium-16), a vinyl pyrrolidone-alkylamino (meth)acrylate copolymer, a vinyl pyrrolidone-alkylamino (meth)acrylate-vinyl caprolactam copolymer, an alkyl acrylamide-(meth)acrylate-alkylamino alkylacrylamide-polyethylene glycol (meth)acrylate copolymer, and an adipic acid-dimethylaminohydroxypropyl ethylenetriamine copolymer, more preferably one or more selected from the group consisting of a quaternized dialkylaminoalkyl (meth)acrylate polymer, a diallyl quaternized ammonium salt polymer, a methacrylamidopropyl trimethylammonium salt polymer, a methacryloyl ethyl trimethylammonium salt polymer, and a vinyl imidazolium trichloride-vinyl pyrrolidone copolymer (polyquaternium-16), still more preferably one or more selected from the group consisting of a diallyl quaternized ammonium salt polymer, a methacrylamidopropyl trimethylammonium salt polymer, and a vinyl imidazolium trichloride-vinyl pyrrolidone copolymer (polyquaternium-16), and even more preferably one or more selected from the group consisting of a dimethyldiallylammonium chloride polymer (polyquaternium-6), a dimethyldiallylammonium chloride-acrylic acid copolymer (polyquaternium-22), an acrylamide-acrylic acid-dimethyldiallylammonium chloride copolymer (polyquaternium-39), an acrylic acid-methyl acrylate-methacrylamidopropyl trimethylammonium chloride copolymer (polyquaternium-47), and a vinyl imidazolium trichloride-vinyl pyrrolidone copolymer (polyquaternium-16).
<10> The method for producing an emulsion composition according to any one of <1> to <9>, in which the component (A) is even more preferably one or more selected from the group consisting of a dimethyldiallylammonium chloride polymer (polyquaternium-6), an acrylamide-acrylic acid-dimethyldiallylammonium chloride copolymer (polyquaternium-39), and a vinylimidazolium trichloride-vinylpyrrolidone copolymer (polyquaternium-16).
<11> The method for producing an emulsion composition according to any one of <3>, <9>, and <10>, in which the cationic charge density of the dimethyldiallylammonium chloride polymer (polyquaternium-6) is even more preferably 2.0 meq/g or more, and even more preferably 2.5 meq/g or more, and is even more preferably 6.5 meq/g or less, and the weight average molecular weight is still more preferably 10,000 or more and 200,000 or less.
<12> The method for producing an emulsion composition according to <3> or <9>, in which the cationic charge density of the dimethyldiallylammonium chloride-acrylic acid copolymer (polyquaternium-22) is even more preferably 2.0 meq/g or more, even more preferably 3.0 meq/g or more, and even more preferably 4.0 meq/g or more, and is even more preferably 6.5 meq/g or less and even more preferably 6.0 meq/g or less, and the weight average molecular weight is still more preferably 20,000 or more, even more preferably 50,000 or more, even more preferably 100,000 or more, even more preferably 200,000 or more, and even more preferably 300,000 or more, and is still more preferably 1,000,000 or less, even more preferably 600,000 or less, and even more preferably 500,000 or less.
<13> The method for producing an emulsion composition according to any one of <3>, <9>, and <10>, in which the cationic charge density of the acrylamide-acrylic acid-dimethyldiallylammonium chloride copolymer (polyquaternium-39) is even more preferably 2.0 meq/g or more, and even more preferably 2.5 meq/g or more, and is even more preferably 6.5 meq/g or less, even more preferably 5.0 meq/g or less, and even more preferably 4.0 meq/g or less, and the weight average molecular weight is still more preferably 20,000 or more, even more preferably 50,000 or more, and even more preferably 100,000 or more, and is still more preferably 1,000,000 or less, even more preferably 600,000 or less, even more preferably 500,000 or less, and even more preferably 300,000 or less.
<14> The method for producing an emulsion composition according to <3> or <9>, in which the cationic charge density of the acrylic acid-methyl acrylate-methacrylamidopropyl trimethylammonium chloride copolymer (polyquaternium-47) is even more preferably 2.0 meq/g or more, and even more preferably 2.5 meq/g or more, and is even more preferably 6.5 meq/g or less, even more preferably 5.0 meq/g or less, and even more preferably 4.5 meq/g or less, and the weight average molecular weight is still more preferably 200,000 or more, even more preferably 500,000 or more, and even more preferably 1,000,000 or more, and is preferably 5,000,000 or less, more preferably 3,000,000 or less, and still more preferably 1,500,000 or less.
<15> The method for producing an emulsion composition according to any one of <3>, <9>, and <10>, in which the cationic charge density of the vinyl imidazolium trichloride-vinyl pyrrolidone copolymer (polyquaternium-16) is even more preferably 2.0 meq/g or more, and is even more preferably 6.5 meq/g or less, even more preferably 5.0 meq/g or less, and even more preferably 4.5 meq/g or less, and the weight average molecular weight is still more preferably 20,000 or more, and even more preferably 50,000 or more, and is still more preferably 150,000 or less.
<16> The method for producing an emulsion composition according to any one of <1>, <2>, and <4> to <15>, in which the component (B) is a polymer having an anionic group and substantially having no cationic group and amphoteric group.
<17> The method for producing an emulsion composition according to any one of <1>, <2>, and <4> to <16>, in which the component (B) is a polymer having an anionic group and a molar amount of a cationic group and an amphoteric group with respect to the anionic group is preferably 0.1% or less.
<18> The method for producing an emulsion composition according to any one of <1>, <2>, and <4> to <17>, in which the anionic group of the component (B) is preferably one or more acidic groups selected from the group consisting of a carboxy group, a sulfonic acid group, and a phosphoric acid group, and more preferably a carboxy group.
<19> The method for producing an emulsion composition according to any one of <1> to <18>, in which an anionic charge density of the component (B) is preferably 0.1 meq/g or more and 30 meq/g or less, more preferably 0.5 meq/g or more and 30 meq/g or less, still more preferably 1.0 meq/g or more and 20 meq/g or less, and even more preferably 2.0 meq/g or more and 15 meq/g or less.
<20> The method for producing an emulsion composition according to any one of <1> to <19>, in which a weight average molecular weight of the component (B) is preferably 3,000 or more and 50,000 or less, more preferably 5,000 or more and 40,000 or less, and still more preferably 10,000 or more and 30,000 or less.
<21> The method for producing an emulsion composition according to any one of <1>, <2>, and <4> to <20>,in which the component (B) is preferably a polymer having a carboxy group, more preferably a carboxy group-containing cellulose derivative, a carboxyvinyl polymer, a poly(meth)acrylic acid, a (meth)acrylic acid-based copolymer, or a salt thereof, still more preferably one or more selected from the group consisting of a poly(meth)acrylic acid, a (meth)acrylic acid-based copolymer, or a salt thereof, even more preferably one or more selected from the group consisting of a polyacrylic acid, a (meth)acrylic acid/alkyl (meth)acrylate copolymer, or a salt thereof, and even more preferably one or more selected from the group consisting of a polyacrylic acid, and an (acrylic acid/stearyl acrylate) copolymer, or a salt thereof.
<22> The method for producing an emulsion composition according to any one of <1> to <21>, in which the component (C) is a compound having a molecular weight of preferably 500 or less and more preferably 200 or less.
<23> The method for producing an emulsion composition according to any one of <1> to <22>, in which the component (C) is one or more selected from the group consisting of a carboxylic acid-based compound and a sulfonic acid-based compound other than the component (B), preferably one or more selected from the group consisting of an aliphatic dicarboxylic acid, hydroxycarboxylic acid, and an aromatic sulfonic acid, more preferably one or more selected from the group consisting of succinic acid, lactic acid, malic acid, glycolic acid, p-toluenesulfonic acid, and naphthalenesulfonic acid, still more preferably one or more selected from the group consisting of succinic acid and lactic acid, and even more preferably lactic acid.
<24> The method for producing an emulsion composition according to any one of <1> to <23>, in which a proportion of the organic acid salt in the component (C) is preferably 70% by mass or less, more preferably 50% by mass or less, still more preferably 35% by mass or less, even more preferably 20% by mass or less, even more preferably 10% by mass or less, even more preferably 5% by mass or less, and even more preferably 2% by mass or less.
<25> The method for producing an emulsion composition according to any one of <1> and <4> to <24>, in which the emulsion composition contains, as an emulsion forming component, preferably component (E): a cationic surfactant, and component (F): a higher alcohol.
<26> The method for producing an emulsion composition according to any one of <2>, <3>, and <25>, in which the component (E) is one or more selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and a salt thereof, (vi) an alkoxyalkyldimethylamine and a salt thereof, and (vii) an alkylamidoalkyldimethylamine and a salt thereof, preferably one or more selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and a salt thereof, (vi) an alkoxyalkyldimethylamine and a salt thereof, and (vii) an alkylamidoalkyldimethylamine and a salt thereof, more preferably one or more selected from the group consisting of (ii) an alkoxyalkyltrimethylammonium and (v) an alkyldimethylamine and a salt thereof, still more preferably one or more selected from the group consisting of stearoxypropyltrimethylammonium chloride, stearoxyethyltrimethylammonium chloride, stearoxyhydroxypropyltrimethylammonium chloride, N,N-dimethyl-3-hexadecyloxypropylamine or a lactic acid salt thereof, and N,N-dimethyl-3-octadecyloxypropylamine (stearoxypropyldimethylamine) or a lactic acid salt thereof, and even more preferably one or more selected from the group consisting of stearoxypropyltrimethylammonium chloride and N,N-dimethyl-3-octadecyloxypropylamine (stearoxypropyldimethylamine) or a lactic acid salt thereof.
<27> The method for producing an emulsion composition according to any one of <2>, <3>, <25>, and <26>, in which the component (F) is an aliphatic monohydric alcohol having 12 or more carbon atoms, preferably 12 or more and 22 or less carbon atoms, more preferably one or more selected from the group consisting of lauryl alcohol, cetyl alcohol, myristyl alcohol, oleyl alcohol, stearyl alcohol, isostearyl alcohol, 2-octyldodecanol, behenyl alcohol, and cetostearyl alcohol, still more preferably one or more selected from the group consisting of cetyl alcohol, myristyl alcohol, stearyl alcohol, and behenyl alcohol, and even more preferably stearyl alcohol.
<28> The method for producing an emulsion composition according to any one of <1> to <27>, in which a blending amount of the component (A) in the mixture A is an amount of preferably 0.01% by mass or more and less than 10% by mass, more preferably 0.03% by mass or more and less than 5% by mass, still more preferably 0.03% by mass or more and less than 1% by mass, and even more preferably 0.05% by mass or more and less than 0.5% by mass, based on a total amount of 100% by mass of the mixture A.
<29> The method for producing an emulsion composition according to any one of <1> to <28>, in which a blending amount of the component (B) in the mixture A is an amount of preferably 0.01% by mass or more and less than 5% by mass, more preferably 0.01% by mass or more and less than 3% by mass, still more preferably 0.02% by mass or more and less than 1% by mass, even more preferably 0.02% by mass or more and less than 0.3% by mass, and even more preferably 0.05% by mass or more and less than 0.3% by mass, based on a total amount of 100% by mass of the mixture A.
<30> The method for producing an emulsion composition according to any one of <1>, and <4> to <29>, in which a blending amount of the component (C) in the mixture A is an amount of preferably 0.01% by mass or more and less than 10% by mass, more preferably 0.01% by mass or more and less than 6% by mass, still more preferably 0.03% by mass or more and less than 1% by mass, even more preferably 0.05% by mass or more and less than 0.75% by mass, and even more preferably 0.05% by mass or more and less than 0.30% by mass, based on a total amount of 100% by mass of the mixture A.
<31> The method for producing an emulsion composition according to any one of <1>, and <4> to <30>, in which in the mixture A, a mass ratio [(A + B)/C] of the total blending amount of the component (A) and the component (B) to the blending amount of the component (C) is in a range of preferably 1/0.10 to 1/20, more preferably 1/0.15 to 1/15, still more preferably 1/0.20 to 1/10, even more preferably 1/0.20 to 1/8, and even more preferably 1/0.22 to 1/4.
<32> The method for producing an emulsion composition according to any one of <1> to <31>, in which a blending amount of the component (D) in the mixture A is an amount of preferably 50% by mass or more and 99.97% by mass or less, more preferably 50% by mass or more and 99.8% by mass or less, still more preferably 60% by mass or more and 99.8% by mass or less, even more preferably 70% by mass or more and 99.8% by mass or less, and even more preferably 80% by mass or more and 99.8% by mass or less, based on a total amount of 100% by mass of the mixture A.
<33> The method for producing an emulsion composition according to any one of <1> to <32>, in which a total content of the components (A) to (D) in the mixture A is preferably 70% by mass or more, more preferably 80% by mass or more, still more preferably 90% by mass or more, and even more preferably 95% by mass or more, and is 100% by mass or less.
<34> The method for producing an emulsion composition according to any one of <1> to <33>, in which a blending amount of the component (A) in the emulsion composition is an amount of preferably 0.01% by mass or more and less than 0.50% by mass, more preferably 0.02% by mass or more and 0.30% by mass or less, and still more preferably 0.03% by mass or more and 0.20% by mass or less, based on a total amount of 100% by mass of the emulsion composition.
<35> The method for producing an emulsion composition according to any one of <1> to <34>, in which a blending amount of the component (B) in the emulsion composition is an amount of preferably 0.01% by mass or more and less than 0.50% by mass, more preferably 0.010% by mass or more and 0.30% by mass or less, still more preferably 0.015% by mass or more and 0.20% by mass or less, even more preferably 0.015% by mass or more and 0.15% by mass or less, and even more preferably 0.015% by mass or more and 0.10% by mass or less, based on a total amount of 100% by mass of the emulsion composition.
<36> The method for producing an emulsion composition according to any one of <1> to <35>, in which a total blending amount of the component (A) and the component (B) in the emulsion composition is less than 1.0% by mass, preferably 0.01% by mass or more and 0.9% by mass or less, more preferably 0.03% by mass or more and 0.9% by mass or less, still more preferably 0.05% by mass or more and 0.9% by mass or less, even more preferably 0.05% by mass or more and 0.5% by mass or less, and even more preferably 0.05% by mass or more and 0.3% by mass or less, based on a total amount of 100% by mass of the emulsion composition.
<37> The method for producing an emulsion composition according to any one of <1>, and <4> to <35>, in which a ratio of number of moles of cationic charges of the component (A) to number of moles of anionic charges of the component (B) in the emulsion composition is in an amount of preferably 5/95 to 95/5, more preferably 10/90 to 90/10, still more preferably 20/80 to 80/20, even more preferably 20/80 to 60/40, and even more preferably 40/60 to 60/40.
<38> The method for producing an emulsion composition according to any one of <1> to <37>, in which a blending amount of the component (C) in the emulsion composition is an amount of preferably 0.01% by mass or more and 5.0% by mass or less, more preferably 0.02% by mass or more and 5.0% by mass or less, still more preferably 0.03% by mass or more and 5.0% by mass or less, even more preferably 0.05% by mass or more and 3.0% by mass or less, even more preferably 0.1% by mass or more and 2.0% by mass or less, even more preferably 0.2% by mass or more and 1.5% by mass or less, and even more preferably 0.3% by mass or more and 1.2% by mass or less, based on a total amount of 100% by mass of the emulsion composition.
<39> The method for producing an emulsion composition according to any one of <1> to <38>, in which a blending amount of the component (D) in the emulsion composition is an amount of preferably 10% by mass or more and 99% by mass or less, more preferably 20% by mass or more and 99% by mass or less, still more preferably 30% by mass or more and 99% by mass or less, even more preferably 40% by mass or more and 95% by mass or less, even more preferably 50% by mass or more and 95% by mass or less, even more preferably 60% by mass or more and 95% by mass or less, even more preferably 70% by mass or more and 95% by mass or less, and even more preferably 80% by mass or more and 90% by mass or less, based on a total amount of 100% by mass of the emulsion composition.
<40> The method for producing an emulsion composition according to any one of <2>, <3>, and <25> to <39>, in which a blending amount of the component (E) in the emulsion composition is an amount of preferably 0.1% by mass or more and 10% by mass or less, more preferably 0.2% by mass or more and 5.0% by mass or less, still more preferably 0.5% by mass or more and 5.0% by mass or less, and even more preferably 1.0% by mass or more and 3.5% by mass or less.
<41> The method for producing an emulsion composition according to any one of <2>, <3>, and <25> to <40>, in which a blending amount of the component (F) in the emulsion composition is an amount of preferably 0.1% by mass or more and 15% by mass or less, more preferably 0.2% by mass or more and 12% by mass or less, still more preferably 0.5% by mass or more and 12% by mass or less, and even more preferably 1.0% by mass or more and 10% by mass or less.
<42> The method for producing an emulsion composition according to any one of <1> to <41>, in which the emulsion composition further contains component (G): an oil agent.
<43> The method for producing an emulsion composition according to <42>, in which the component (G) is one or more selected from the group consisting of (a) silicone oil, (b) ester oil, (c) ether oil, (d) hydrocarbon oil, (e) higher fatty acid, and (f) wax, preferably one or more selected from the group consisting of (a) silicone oil, (b) ester oil, (d) hydrocarbon oil, and (e) higher fatty acid, and more preferably containing one or more selected from the group consisting of (a) silicone oil and (e) higher fatty acid, still more preferably containing one or more selected from the group consisting of dimethylpolysiloxane, dimethiconol, methylphenylpolysiloxane, modified silicone, and fatty acids having 12 or more carbon atoms, even more preferably containing one or more selected from the group consisting of dimethylpolysiloxane, dimethiconol, amino-modified silicone, polyether-modified silicone, aminopolyether-modified silicone, lauric acid, myristic acid, palmitic acid, stearic acid, behenic acid, oleic acid, coconut oil fatty acids, lanolin fatty acids, isostearyl acid, and isopalmitic acid, even more preferably containing one or more selected from the group consisting of dimethylpolysiloxane, amino-modified silicone, aminopolyether-modified silicone, and lanolin fatty acids, and even more preferably containing dimethylpolysiloxane, amino-modified silicone, and lanolin fatty acids.
<44> The method for producing an emulsion composition according to <42> or <43>, in which a blending amount of the component (G) in the emulsion composition is an amount of preferably 0.1% by mass or more and 20% by mass or less, more preferably 0.2% by mass or more and 10% by mass or less, still more preferably 0.5% by mass or more and 10% by mass or less, and even more preferably 1.0% by mass or more and 8.0% by mass or less.
<45> The method for producing an emulsion composition according to any one of <1> to <44>, in which the emulsion composition further contains component (H): an aqueous medium.
<46> The method for producing an emulsion composition according to <45>, in which the component (H) is one or more selected from the group consisting of lower alcohols, low-molecular-weight diols and triols having 6 or less carbon atoms, and preferably one or more selected from the group consisting of ethanol, isopropyl alcohol, 1,3-butylene glycol, glycerin, ethylene glycol, propylene glycol, and dipropylene glycol.
<47> The method for producing an emulsion composition according to <45> or <46>, in which a blending amount of the component (H) in the emulsion composition is an amount of preferably 0.1% by mass or more and 10% by mass or less, more preferably 0.2% by mass or more and 5.0% by mass or less, still more preferably 0.5% by mass or more and 5.0% by mass or less, and even more preferably 1.0% by mass or more and 3.0% by mass or less.
<48> The method for producing an emulsion composition according to any one of <1> to <47>, in which the pH of a 5% aqueous solution of the emulsion composition at 25°C is preferably 2.0 or more, and more preferably 3.0 or more, and is preferably 10.0 or less, more preferably 7.0 or less, and still more preferably 5.0 or less.
<49> The method for producing an emulsion composition according to any one of <1> to <48>, in which the temperature at the time of mixing of the components (A) to (D) in the step (1) is preferably 40°C or higher and 90°C or lower, more preferably 45°C or higher and 80°C or lower, still more preferably 50°C or higher and 70°C or lower, and even more preferably 50°C or higher and 65°C or lower.
<50> The method for producing an emulsion composition according to any one of <1> to <49>, in which the mixing time of the components (A) to (D) in the step (1) is preferably 1 minute or more, more preferably 3 minutes or more, and is preferably 1 hour or less.
<51> The method for producing an emulsion composition according to any one of <1> to <50>, in which in the step (1), the component (A) and the component (C) are added to the component (D) and mixed, and then the component (B) is added and mixed.
<52> The method for producing an emulsion composition according to any one of <1> to <51>, in which the mixture A obtained in the step (1) is in a state of a suspension.
<53> The method for producing an emulsion composition according to any one of <1> to <52>, in which the pH of the mixture A obtained in the step (1) at 25°C is preferably 1.5 or more and 4.0 or less, more preferably 2.0 or more and 4.0 or less, still more preferably 2.0 or more and 3.2 or less, and even more preferably 2.0 or more and 3.0 or less.
<54> The method for producing an emulsion composition according to any one of <1> and <4> to <53>, in which the temperature at the time of mixing of the mixture A and the oil phase component containing the emulsion forming component in the step (2-1) is preferably 40°C or higher and 90°C or lower, more preferably 45°C or higher and 80°C or lower, and still more preferably 50°C or higher and 70°C or lower.
<55> The method for producing an emulsion composition according to any one of <1> to <54>, in which the mixing time of the mixture A and the oil phase component containing the emulsion forming component in the step (2-1) is preferably 1 minute or more, more preferably 3 minutes or more, and is preferably 1 hour or less.
<56> The method for producing an emulsion composition according to any one of <1> to <55>, in which the temperature at the time of mixing of the oil phase component containing the emulsion forming component and the aqueous phase component other than the mixture A in the step (2-2) is preferably 40°C or higher and 90°C or lower, more preferably 45°C or higher and 90°C or lower, and still more preferably 50°C or higher and 80°C or lower.
<57> The method for producing an emulsion composition according to any one of <1> to <56>, in which the mixing time of the oil phase component containing the emulsion forming component and the aqueous phase component other than the mixture A in the step (2-2) is preferably 1 minute or more, more preferably 3 minutes or more, and is preferably 1 hour or less.
<58> The method for producing an emulsion composition according to any one of <1> and <4> to <57>, in which the temperature at the time of mixing of the emulsion (II) and the mixture A in the step (2-2) is preferably 10°C or higher and 50°C or lower, and more preferably 20°C or higher and 45°C or lower.
<59> The method for producing an emulsion composition according to any one of <1> to <58>, in which the mixing time of the emulsion (II) and the mixture A in the step (2-2) is preferably 1 minute or more, more preferably 3 minutes or more, and from the viewpoint of productivity, is preferably 1 hour or less.
<60> The method for producing an emulsion composition according to any one of <1> to <59>, including the step (1) and the step (2-1).
<61> A method for improving the stability of an emulsion composition, including the method for producing an emulsion composition according to any one of <1> to <60>.
<62> A method for suppressing spread of hair under high humidity conditions, including a step of applying the emulsion composition according to any one of <1> to <60> to hair.
<63> A method for improving stability of an emulsion composition containing
   component (A): one or more polymers selected from the group consisting of cationic polymers and amphoteric polymers; and
   component (B): an anionic polymer,
   the method including the following step (1) and the following step (2-1) or step (2-2),
   in which a total blending amount of the component (A) and the component (B) in the emulsion composition is less than 1.0% by mass based on 100% by mass of the total amount of the emulsion composition:
      step (1): a step for preparing a mixture A containing the component (A), the component (B), component (C): an organic acid, and component (D): water;
      step (2-1): a step for preparing an emulsion (I) by mixing the mixture A with an oil phase component containing an emulsion forming component;
      step (2-2): a step for preparing an emulsion (II) by mixing an oil phase component containing an emulsion forming component with an aqueous phase component other than the mixture A, followed by mixing with the mixture A.

### Examples

Hereinafter, the present invention will be described by Examples, but the present invention is not limited to the scope of the Examples. The measurements and evaluations in Examples were performed by the following methods.

### (pH Measurement)

For the emulsion composition (hair conditioner) of each example, water was added to the emulsion composition to obtain a 5% aqueous solution, and the pH of the 5% aqueous solution at 25°C was measured using a pH meter (F-51, manufactured by HORIBA, Ltd.). The pH was 3.4 in all cases.

In addition, for the mixture A or A, the pH of the mixture A or A' at 25°C was measured using the above-mentioned pH meter. The mixture prepared in the step (1) and not corresponding to the "mixture A" defined in the present invention is referred to as "mixture A‴.

### (Emulsified State)

One g of the hair conditioner of each example was dropped on a black plastic underlay, and the appearance and feel when the surface was traced with a finger 10 times were evaluated according to the following criteria.
A: Aggregated particles are not visually recognized, and a smooth feel is felt when the surface is traced with a finger.
B: Aggregated particles are not visually recognized, but a rough feel is felt when the surface is traced with a finger.
C: Aggregated particles are visually recognized, and a rough feel is felt when the surface is traced with a finger.
D: Separated

### (Storage Stability)

The storage stability of the hair conditioner of each example was evaluated by a cycle test.

A cycle test was conducted in which the hair conditioner of each example was stored for two weeks in an environment in which the temperature changes in the range of 40°C to -28°C. The temperature cycle per day was set so that the cycle of the temperature difference ΔT = 40°C was four laps/day. The presence or absence of separation of the hair conditioner after storage was visually observed, and in the Tables, the case where there was no separation and it was stable was indicated by "A", and the case where separation was recognized was indicated by "D".

### (Feel at the Time of Application)

A bleaching agent (a mixture of "Kao Foam Hair Bleach L" (first liquid) 7 mL and "Kao Foam Hair Color d" (second liquid) 14 mL) was applied to a tress of Japanese human hair with a length of 30 cm and a mass of 20 g, allowed to stand for 20 minutes, and then sufficiently rinsed with warm water at 35 to 40°C. The above treatment was repeated four times to prepare a tress for evaluation of Japanese damaged hair.

1 mL of the hair conditioner of each example was applied to the above-described tress for evaluation, and the hair conditioner was applied to the tress by hand. The feel at that time was evaluated in five grades by five professional panelists according to the following criteria, and the average value was taken as the evaluation result.
5: Very smooth
4: Smooth
3: Slightly smooth
2: Smoothness equivalent to that of the case of using a plain conditioner having the following composition.
1: The presence is immediately lost and the feel is not smooth.

### [Plain Conditioner Composition]

(% by mass)
Stearoxypropyldimethylamine (*1) 0.9
Stearyl alcohol (*2) 3.0
Dipropylene glycol 0.5
Benzyl alcohol 0.2
Lactic acid (*3) 1.1
Purified water Remaining amount
Total 100.0
(*1) "FARMIN DM-E80" manufactured by Kao Corporation, active ingredient amount: 90% by mass
(*2) "KALCOL 8098" manufactured by Kao Corporation, active ingredient amount: 100% by mass
(*3) "PURAC ULTRAPURE 90" manufactured by PURAC Tiland Ltd., active ingredient amount: 90% by mass

### (Moisture Resistance of Hair after Treatment)

One mL of the hair conditioner of each example was applied to the tress for evaluation of Japanese damaged hair produced by the method described above, and the tress was allowed to stand for 30 seconds. After being left for 1 minute in this state, the hair was rinsed with running water for 30 seconds and dried with a dryer until completely dried.

The tress after drying was subjected to 10 times of straight setting while passing through a flat iron ("AHI-938" manufactured by Miki Denki Sangyo Co., Ltd.) set to 160°C using a comb, and a photograph of the tress immediately after setting was taken. Next, the hair after the straight setting was suspended and left to stand for 30 minutes in a high humidity environment of 35°C and 80% RH, and a photograph was taken again. In the two photographs, the width of the tress at a position of 23.5 cm from the root of the tress was measured, and the change in the width of the tress immediately after straight setting and after storage under high humidity was calculated by the following formula. Tress width change value = (tress width after storage under high humidity)/(tress width immediately after straight setting)

When the above value exceeds 1.3, it is visually recognized that the tress is spread, and thus it is determined that the moisture resistance is low.

### Example 1 (Production of Hair Conditioner)

### <Step (1)>

Mixture A was prepared by the following procedure using the components described in Table 1.

In a 500 mL beaker, 1.52 g of a polyquaternium-6 solution as the component (A), 1 g of lactic acid (90% by mass) as the component (C), and 318 g of water (corresponding to 63.59% by mass) as the component (D) were charged, and heated and stirred at 56°C using a stirring device (mechanical stirrer equipped with two propeller blades). After confirming that the mixture became a uniform solution, 0.37 g of sodium polyacrylate as the component (B) was added and mixed to obtain a mixture A as a uniform suspension.

### <Step (2-1)>

Next, an emulsion composition (hair conditioner) was prepared by the following procedure.

First, 22.22 g of stearyloxypropyltrimethylammonium chloride as the component (E), 10.19 g of stearyl alcohol as the component (F), and 10.0 g of dipropylene glycol described in Table 1 were mixed in advance, and heated and dissolved at 85°C to prepare an oil phase component containing an emulsion forming component. This was added to the mixture A, and heated and stirred at 56°C for 10 minutes. After stirring for 10 minutes, heating was terminated and air cooling was started (preparation of emulsion (I)).

Next, the components (G) (1.0 g of amodimethicone and 10.0 g of dimethicone) described in Table 1 were added and mixed.

Further, 120 g of water (corresponding to 24.00% by mass) at 30°C, 5 g of lanolin fatty acid, and 0.75 g of lactic acid (90% by mass) were added thereto, and the mixture was cooled with stirring until the liquid temperature became 40°C or lower to obtain 500 mL of a hair conditioner.

The obtained hair conditioner was used to perform evaluation by the above-described methods. The results are shown in Table 1. The blending amounts described in the table of the present examples are the blending amounts (% by mass) of each component as it is, and the "active ingredient concentration" described in the tables of the present examples is the content or blending amount (% by mass) of each component converted into the active ingredient amount.

### Examples 2 to 3, 5 to 9, 11 to 16 and Comparative Examples 2 to 6

Hair conditioners were produced and evaluated in the same manner as in Example 1, except that the types and amounts of the respective components used in the step (1) and the step (2-1) were changed as described in Tables 1 to 3. The results are shown in Tables 1 to 3.

### Example 4

### <Step (1)>

Mixture A was prepared by the following procedure using the components described in Table 1.

Into a 100 mL beaker, 1.52 g of a polyquaternium-6 solution as the component (A), 1 g of lactic acid (90% by mass) as the component (C), and 15 g of water (corresponding to 3.00% by mass) as the component (D) were put and heated and stirred at 56°C using a stirring device (mechanical stirrer equipped with two propeller blades). After confirming that the mixture became a uniform solution, 0.37 g of sodium polyacrylate as the component (B) was added and mixed to obtain a mixture A as a uniform suspension.

### <Step (2-2)>

Next, an emulsion composition (hair conditioner) was prepared by the following procedure.

First, 22.22 g of stearyloxypropyltrimethylammonium chloride as the component (E), 10.19 g of stearyl alcohol as the component (F), and 10.0 g of dipropylene glycol described in Table 1 were mixed in advance, and heated and dissolved at 85°C to prepare an oil phase component containing an emulsion forming component.

Into a 500 mL beaker, 303 g (corresponding to 60.59% by mass) of water was put and heated and stirred at 56°C using a stirring device (mechanical stirrer equipped with two propeller blades). The oil phase components were added thereto, and the mixture was heated and stirred at 56°C for 10 minutes. After stirring for 10 minutes, heating was terminated and air cooling was started (preparation of emulsion (II)). After air cooling, the mixture A was added and stirred for 10 minutes (mixture of the emulsion (II) and the mixture A).

Next, the components (G) (amodimethicone 1.0 g and dimethicone 10.0 g) described in Table 1 were added and mixed, then 120 g (corresponding to 24.00% by mass) of water at 30°C, 5.0 g of lanolin fatty acid, and 0.75 g of lactic acid (90% by mass) were added, and the mixture was cooled while stirring until the liquid temperature became 40°C or lower to obtain 500 mL of a hair conditioner.

The obtained hair conditioner was used to perform evaluation by the above-described methods. The results are shown in Table 1.

### Example 10

### <Step (1)>

Mixture A was prepared by the following procedure using the components described in Table 2.

Into a 500 mL beaker, 1.52 g of a polyquaternium-6 solution as the component (A), 1 g of lactic acid (90% by mass) as the component (C), and 438 g of water (corresponding to 87.59% by mass) as the component (D) were put, and heated and stirred at 75°C using a stirring device (mechanical stirrer equipped with two propeller blades). After confirming that the mixture became a uniform solution, 0.37 g of sodium polyacrylate as the component (B) was added and mixed to obtain a mixture A as a uniform suspension.

### <Step (2-1)>

Next, an emulsion composition (hair conditioner) was prepared by the following procedure.

First, 22.22 g of stearyloxypropyltrimethylammonium chloride as the component (E), 10.19 g of stearyl alcohol as the component (F), and 10.0 g of dipropylene glycol described in Table 2 were mixed in advance, and heated and dissolved at 85°C to prepare an oil phase component containing an emulsion forming component. This was added to the mixture A, and heated and stirred at 75°C for 10 minutes. After stirring for 10 minutes, heating was terminated and air cooling was started (preparation of emulsion (I)).

Next, the components (G) (amodimethicone 1.0 g, dimethicone 10.0 g, lanolin fatty acid 5 g) described in Table 2 were added, and further 0.75 g of lactic acid (90% by mass) was added, and the mixture was cooled with stirring until the liquid temperature became 40°C or lower to obtain 500 mL of a hair conditioner.

The obtained hair conditioner was used to perform evaluation by the above-described methods. The results are shown in Table 2.

### Example 17

### <Step (1)>

Mixture A was prepared by the following procedure using the components described in Table 2.

In a 500 mL beaker, 1.52 g of a polyquaternium-6 solution as the component (A), 3 g of lactic acid (90% by mass) as the component (C), and 422.71 g of water (corresponding to 84.54% by mass) as the component (D) were charged, and heated and stirred at 80°C using a stirring device (mechanical stirrer equipped with two propeller blades). After confirming that the mixture became a uniform solution, 0.37 g of sodium polyacrylate as the component (B) was added and mixed to obtain a mixture A as a uniform suspension.

### <Step (2-1)>

Next, an emulsion composition (hair conditioner) was prepared by the following procedure.

First, 11.00 g of stearoxypropyldimethylamine as the component (E), 31.90 g of stearyl alcohol as the component (F), and 10.0 g of dipropylene glycol described in Table 2 were mixed in advance, and heated and dissolved at 75°C to prepare an oil phase component containing an emulsion forming component. This was added to the mixture A, and heated and stirred at 75°C for 10 minutes. After stirring for 10 minutes, heating was terminated and air cooling was started (preparation of emulsion (I)).

Next, the components (G) (1.0 g of amodimethicone and 10.0 g of dimethicone) described in Table 2 were added and mixed.

Further, 5.0 g of lanolin fatty acid, and 3.5 g of lactic acid (90% by mass) were added thereto, and the mixture was cooled with stirring until the liquid temperature became 40°C or lower to obtain 500 mL of a hair conditioner.

The obtained hair conditioner was used to perform evaluation by the above-described methods. The results are shown in Table 2.

### Comparative Example 1

22.22 g of stearyloxypropyldimethylammonium chloride as the component (E), 10.19 g of stearyl alcohol as the component (F), and 10.0 g of dipropylene glycol described in Table 3 were mixed in advance, and heated and dissolved at 75°C to prepare an oil phase component containing an emulsion forming component.

In a 500 mL beaker, 1.52 g of a polyquaternium-6 solution as the component (A), 1 g of lactic acid (90% by mass) as the component (C), and 318 g of water (corresponding to 63.59% by mass) as the component (D) were charged, and heated and stirred at 56°C using a stirring device (mechanical stirrer equipped with two propeller blades) (preparation of mixture A'). After confirming that the mixture became a homogeneous solution, the oil phase components were added and heated and stirred at 56°C for 10 minutes (mixing of the mixture A' and the oil phase components containing the emulsion forming component). After stirring for 10 minutes, heating was terminated and air cooling was started. Then, 0.37 g of sodium polyacrylate as the component (B) was added thereto and mixed, followed by stirring for 10 minutes.

Next, the components (G) (amodimethicone 1.0 g and dimethicone 10.0 g) described in Table 3 were added and mixed, then 120 g (corresponding to 24.00% by mass) of water at 30°C, 5 g of lanolin fatty acid, and 0.75 g of lactic acid (90% by mass) were added, and the mixture was cooled while stirring until the liquid temperature became 40°C or lower to obtain 500 mL of a hair conditioner.

The obtained hair conditioner was used to perform evaluation by the above-described methods. The results are shown in Table 3.

**Table 1**

| (As it is: % by mass) | | | | Example 1 | Example 2 | Example 3 | Example 4 | Example 5 | Example 6 | Example 7 | Example 8 | Example 9 |
|---|---|---|---|---|---|---|---|---|---|---|---|---|
| Blending component of Step (1) | Mixture A or A' | (A) | Polyquatemium-6 *1 | 0.304 | 0.304 | 0.304 | 0.304 | | | | | 0.289 |
| | | | Polyquatemium-39 *2 | | | | | 0.363 | | | | |
| | | | Polyquatemium-16 *3 | | | | | | 0.365 | | | |
| | | | Polyquatemium-22 *4 | | | | | | | 0.332 | | |
| | | | Polyquatemium-47 *5 | | | | | | | | 0.733 | |
| | | (B) | Sodium polyacrylate *6 | 0.074 | 0.074 | 0.074 | 0.074 | 0.044 | 0.054 | 0.064 | 0.046 | |
| | | | (Acrylic acid/stearyl acrylate) copolymer *7 | | | | | | | | | 0.80 |
| | | (C) | Lactic acid *8 | 0.20 | 0.05 | 0.35 | 0.20 | 0.20 | 0.20 | 0.20 | 0.20 | 0.20 |
| | | (C') | Sodium chloride | | | | | | | | | |
| | | (D) | Water | 63.59 | 63.59 | 63.59 | 3.00 | 63.56 | 63.55 | 63.57 | 63.19 | 62.88 |
| Blending component of Step (2) | Oil phase component containing emulsion forming component | (E) | Stearoxypropyltrimethylammonium chloride *9 | 4.44 | 4.44 | 4.44 | 4.44 | 4.44 | 4.44 | 4.44 | 4.44 | 4.44 |
| | | | Stearoxypropyldimethylamine *10 | | | | | | | | | |
| | | (F) | Stearyl alcohol *11 | 2.04 | 2.04 | 2.04 | 2.04 | 2.04 | 2.04 | 2.04 | 2.04 | 2.04 |
| | | (H) | Dipropylene glycol *12 | 2.00 | 2.00 | 2.00 | 2.00 | 2.00 | 2.00 | 2.00 | 2.00 | 2.00 |
| | Aqueous phase component other than mixture A or A' | (C) | Lactic acid *8 | 0.15 | 0.30 | | 0.15 | 0.15 | 0.15 | 0.15 | 0.15 | 0.15 |
| | | (D) | Water | 24.00 | 24.00 | 24.00 | 84.59 | 24.00 | 24.00 | 24.00 | 24.00 | 24.00 |
| | Other oil phase component | (G) | Dimethicone *13 | 2.00 | 2.00 | 2.00 | 2.00 | 2.00 | 2.00 | 2.00 | 2.00 | 2.00 |
| | | | Amodimethicone *14 | 0.20 | 0.20 | 0.20 | 0.20 | 0.20 | 0.20 | 0.20 | 0.20 | 0.20 |
| | | | Lanolin fatty acid *15 | 1.00 | 1.00 | 1.00 | 1.00 | 1.00 | 1.00 | 1.00 | 1.00 | 1.00 |
| Total | | | | 100.00 | 100.00 | 100.00 | 100.00 | 100.00 | 100.00 | 100.00 | 100.00 | 100.00 |
| Component blending amount in mixture A or A' (Active ingredient concentration) (% by mass) | | | Component (A) | 0.20 | 0.20 | 0.20 | 3.53 | 0.24 | 0.25 | 0.21 | 0.24 | 0.19 |
| | | | Component (B) | 0.12 | 0.12 | 0.12 | 2.07 | 0.07 | 0.08 | 0.10 | 0.07 | 0.12 |
| | | | Component (C) | 0.28 | 0.07 | 0.49 | 5.03 | 0.28 | 0.28 | 0.28 | 0.28 | 0.28 |
| | | | Component (D) | 99.41 | 99.62 | 99.20 | 89.38 | 99.41 | 99.39 | 99.41 | 99.41 | 99.41 |
| pH of mixture A or A' | | | | 2.7 | 3.0 | 2.5 | - | - | - | - | - | - |

**Table 1 (cont'd.)**

| | | Example 1 | Example 2 | Example 3 | Example 4 | Example 5 | Example 6 | Example 7 | Example 8 | Example 9 |
|---|---|---|---|---|---|---|---|---|---|---|
| Production step | Mixture (A or A') prepared in step (1) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) |
| | Appearance of mixture (A or A') prepared in step (1) | Suspension | Suspension | Suspension | Suspension | Suspension | Suspension | Suspension | Suspension | Suspension |
| | Step (2) | (2-1) | (2-1) | (2-1) | (2-2) | (2-1) | (2-1) | (2-1) | (2-1) | (2-1) |
| | Temperature at the mixing in step (2) | 56°C | 56°C | 56°C | 56°C | 56°C | 56°C | 56°C | 56°C | 56°C |
| | Blending amount of component (A) (active ingredient concentration) (% by mass) | 0.126 | 0.126 | 0.126 | 0.126 | 0.156 | 0.157 | 0.136 | 0.154 | 0.120 |
| | Blending amount of component (B) (active ingredient concentration) (% by mass) | 0.074 | 0.074 | 0.074 | 0.074 | 0.044 | 0.054 | 0.064 | 0.046 | 0.080 |
| | Amount of component (C) to be blended in mixture A or A' (active ingredient concentration) (% by mass) | 0.18 | 0.05 | 0.32 | 0.18 | 0.18 | 0.18 | 0.18 | 0.18 | 0.18 |
| | Blending amount of component (C) (active ingredient concentration) (% by mass) | 0.32 | 0.32 | 0.32 | 0.32 | 0.32 | 0.32 | 0.32 | 0.32 | 0.32 |
| | Blending amount of component (D) (% by mass) | 87.59 | 87.59 | 87.59 | 87.59 | 87.56 | 87.55 | 87.57 | 87.19 | 86.88 |
| | Blending amount of component (E) (active ingredient concentration) (% by mass) | 2.00 | 2.00 | 2.00 | 2.00 | 2.00 | 2.00 | 2.00 | 2.00 | 2.00 |
| Formulation of emulsion composition | Blending amount of component (F) (active ingredient concentration) (% by mass) | 4.48 | 4.48 | 4.48 | 4.48 | 4.48 | 4.48 | 4.48 | 4.48 | 4.48 |
| | Blending amount of component (G) (active ingredient concentration) (% by mass) | 2.24 | 2.24 | 2.24 | 2.24 | 2.24 | 2.24 | 2.24 | 2.24 | 2.24 |
| | Blending amount of component (H) (active ingredient concentration) (% by mass) | 2.00 | 2.00 | 2.00 | 2.00 | 2.00 | 2.00 | 2.00 | 2.00 | 2.00 |
| | Total blending amount of components (A) + (B) (active ingredient concentration) (% by mass) | 0.20 | 0.20 | 0.20 | 0.20 | 0.20 | 0.21 | 0.20 | 0.20 | 0.20 |
| | Cationic charges of component (A)/Anionic charges of component (B) molar ratio | 50/50 | 50/50 | 50/50 | 50/50 | 50/50 | 50/50 | 50/50 | 50/50 | 50/50 |
| | Blending ratio in mixture A or A' [(A+B)/C] | 1/0.9 | 1/0.25 | 1/1.6 | 1/0.9 | 1/0.9 | 1/0.9 | 1/0.9 | 1/0.9 | 1/0.9 |
| Evaluation results | Emulsified state | A | A | B | C | B | A | B | A | A |
| | Storage stability | A | A | A | A | A | A | A | A | A |
| | Feel at the time of application | 4.2 | 4.4 | 3.6 | 3.2 | 4.8 | 4.0 | 3.8 | 3.8 | 3.6 |
| | Moisture resistance of hair after treatment (tress width change value after storage under high humidity condition) | 1.0 | 1.0 | 1.2 | 1.2 | 1.2 | 1.0 | 1.0 | 1.0 | 1.1 |

**Table 2**

| (As it is: % by mass) | | | | Example 10 | Example 11 | Example 12 | Example 13 | Example 14 | Example 15 | Example 16 | Example 17 |
|---|---|---|---|---|---|---|---|---|---|---|---|
| Blending component of Step (1) | Mixture A or A' | (A) | Polyquatemium-6 *1 | 0.304 | 0.455 | 0.082 | 0.453 | 0.386 | 0.205 | 0.077 | 0.304 |
| | | | Polyquatemium-39 *2 | | | | | | | | |
| | | | Polyquatemium-16 *3 | | | | | | | | |
| | | | Polyquatemium-22 *4 | | | | | | | | |
| | | | Polyquatemium-47 *5 | | | | | | | | |
| | | (B) | Sodium polyacrylate *6 | 0.074 | 0.110 | 0.016 | 0.012 | 0.040 | 0.115 | 0.168 | 0.074 |
| | | | (Acrylic acid/stearyl acrylate) copolymer *7 | | | | | | | | |
| | | (C) | Lactic acid *8 | 0.20 | 0.20 | 0.20 | 0.20 | 0.20 | 0.20 | 0.20 | 0.60 |
| | | (C') | Sodium chloride | | | | | | | | |
| | | (D) | Water | 87.59 | 63.40 | 63.87 | 63.50 | 63.54 | 63.65 | 63.72 | 84.54 |
| Blending component of Step (2) | Oil phase component containing emulsion forming component | (E) | Stearoxypropyltrimethylammonium chloride *9 | 4.44 | 4.44 | 4.44 | 4.44 | 4.44 | 4.44 | 4.44 | |
| | | | Stearoxypropyldimethylamine *10 | | | | | | | | 2.20 |
| | | (F) | Stearyl alcohol *11 | 2.04 | 2.04 | 2.04 | 2.04 | 2.04 | 2.04 | 2.04 | 6.38 |
| | | (H) | Dipropylene glycol *12 | 2.00 | 2.00 | 2.00 | 2.00 | 2.00 | 2.00 | 2.00 | 2.00 |
| | Aqueous phase component other than mixture A or A' | (C) | Lactic acid *8 | 0.15 | 0.15 | 0.15 | 0.15 | 0.15 | 0.15 | 0.15 | 0.70 |
| | | (D) | Water | 0.00 | 24.00 | 24.00 | 24.00 | 24.00 | 24.00 | 24.00 | |
| | Other oil phase component | (G) | Dimethicone * 13 | 2.00 | 2.00 | 2.00 | 2.00 | 2.00 | 2.00 | 2.00 | 2.00 |
| | | | Amodimethicone *14 | 0.20 | 0.20 | 0.20 | 0.20 | 0.20 | 0.20 | 0.20 | 0.20 |
| | | | Lanolin fatty acid * 15 | 1.00 | 1.00 | 1.00 | 1.00 | 1.00 | 1.00 | 1.00 | 1.00 |
| Total | | | | 100.00 | 100.00 | 100.00 | 100.00 | 100.00 | 100.00 | 100.00 | 100.00 |
| Component blending amount in mixture A or A' (Active ingredient concentration) (% by mass) | | | Component (A) | 0.14 | 0.30 | 0.05 | 0.29 | 0.25 | 0.13 | 0.05 | 0.15 |
| | | | Component (B) | 0.08 | 0.17 | 0.02 | 0.02 | 0.06 | 0.18 | 0.26 | 0.09 |
| | | | Component (C) | 0.20 | 0.28 | 0.28 | 0.28 | 0.28 | 0.28 | 0.28 | 0.63 |
| | | | Component (D) | 99.57 | 99.25 | 99.64 | 99.41 | 99.41 | 99.41 | 99.41 | 99.13 |
| pH of mixture A or A' | | | | - | - | - | - | - | - | - | 2.4 |

**Table 2 (cont'd.)**

| | | Example 10 | Example 11 | Example 12 | Example 13 | Example 14 | Example 15 | Example 16 | Example 17 |
|---|---|---|---|---|---|---|---|---|---|
| Production step | Mixture (A or A') prepared in step (1) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) | Mixing of (A), (B), (C) and (D) |
| | Appearance of mixture (A or A') prepared in step (1) | Suspension | Suspension | Suspension | Suspension | Suspension | Suspension | Suspension | Suspension |
| | Step (2) | (2-1) | (2-1) | (2-1) | (2-1) | (2-1) | (2-1) | (2-1) | (2-1) |
| | Temperature at the mixing in step (2) | 75°C | 56°C | 56°C | 56°C | 56°C | 56°C | 56°C | 75°C |
| | Blending amount of component (A) (active ingredient concentration) (% by mass) | 0.126 | 0.189 | 0.034 | 0.188 | 0.160 | 0.085 | 0.032 | 0.126 |
| | Blending amount of component (B) (active ingredient concentration) (% by mass) | 0.074 | 0.110 | 0.016 | 0.012 | 0.040 | 0.115 | 0.168 | 0.074 |
| | Amount of component (C) to be blended in mixture A or A' (active ingredient concentration) (% by mass) | 0.18 | 0.18 | 0.18 | 0.18 | 0.18 | 0.18 | 0.18 | 0.54 |
| | Blending amount of component (C) (active ingredient concentration) (% by mass) | 0.32 | 0.32 | 0.32 | 0.32 | 0.32 | 0.32 | 0.32 | 1.17 |
| | Blending amount of component (D) (% by mass) | 87.59 | 87.40 | 87.87 | 87.50 | 87.54 | 87.65 | 87.72 | 84.54 |
| Formulation of emulsion composition | Blending amount of component (E) (active ingredient concentration) (% by mass) | 2.00 | 2.00 | 2.00 | 2.00 | 2.00 | 2.00 | 2.00 | 1.98 |
| | Blending amount of component (F) (active ingredient concentration) (% by mass) | 4.48 | 4.48 | 4.48 | 4.48 | 4.48 | 4.48 | 4.48 | 6.60 |
| | Blending amount of component (G) (active ingredient concentration) (% by mass) | 2.24 | 2.24 | 2.24 | 2.24 | 2.24 | 2.24 | 2.24 | 2.24 |
| | Blending amount of component (H) (active ingredient concentration) (% by mass) | 2.00 | 2.00 | 2.00 | 2.00 | 2.00 | 2.00 | 2.00 | 2.00 |
| | Total blending amount of components (A) + (B) (active ingredient concentration) (% by mass) | 0.20 | 0.30 | 0.05 | 0.20 | 0.20 | 0.20 | 0.20 | 0.20 |
| | Cationic charges of component (A)/Anionic charges of component (B) molar ratio | 50/50 | 50/50 | 50/50 | 90/10 | 70/30 | 30/70 | 10/90 | 55/45 |
| | Blending ratio in mixture A or A' [(A+B)/C] | 1/0.9 | 1/0.6 | 1/3.6 | 1/0.9 | 1/0.9 | 1/0.9 | 1/0.9 | 1/2.7 |
| Evaluation results | Emulsified state | B | A | A | B | B | A | B | A |
| | Storage stability | A | A | A | A | A | A | A | A |
| | Feel at the time of application | 4 | 4 | 4.4 | 3.4 | 3.6 | 3.8 | 3.8 | 4.2 |
| | Moisture resistance of hair after treatment (tress width change value after storage under high humidity condition) | 1.1 | 1.2 | 1.2 | 1.1 | 1.2 | 1.2 | 1.2 | 1.0 |

**Table 3**

| (As it is: % by mass) | | | | Comparative Example 1 | Comparative Example 2 | Comparative Example 3 | Comparative Example 4 | Comparative Example 5 | Comparative Example 6 |
|---|---|---|---|---|---|---|---|---|---|
| Blending component of Step (1) | Mixture A or A' | (A) | Polyquatemium-6 *1 | 0.304 | 0.304 | 0.304 | | 1.520 | 0.304 |
| | | | Polyquatemium-39 *2 | | | | | | |
| | | | Polyquatemium-16 *3 | | | | | | |
| | | | Polyquatemium-22 *4 | | | | | | |
| | | | Polyquatemium-47 *5 | | | | | | |
| | | (B) | Sodium polyacrylate *6 | 0.074 | 0.074 | | 0.074 | 0.369 | 0.074 |
| | | | (Acrylic acid/stearyl acrylate) copolymer *7 | | | | | | |
| | | (C) | Lactic acid *8 | 0.20 | | 0.20 | 0.20 | 0.20 | |
| | | (C') | Sodium chloride | | | | | | 1.00 |
| | | (D) | Water | 63.59 | 63.59 | 63.67 | 63.90 | 62.08 | 62.59 |
| Blending component of Step (2) | Oil phase component containing emulsion forming component | (E) | Stearoxypropyltrimethylammonium chloride *9 | 4.44 | 4.44 | 4.44 | 4.44 | 4.44 | 4.44 |
| | | | Stearoxypropyldimethylamine *10 | | | | | | |
| | | (F) | Stearyl alcohol *11 | 2.04 | 2.04 | 2.04 | 2.04 | 2.04 | 2.04 |
| | | (H) | Dipropylene glycol *12 | 2.00 | 2.00 | 2.00 | 2.00 | 2.00 | 2.00 |
| | Aqueous phase component other than mixture A or A' | (C) | Lactic acid *8 | 0.15 | 0.35 | 0.15 | 0.15 | 0.15 | 0.35 |
| | | (D) | Water | 24.00 | 24.00 | 24.00 | 24.00 | 24.00 | 24.00 |
| | Other oil phase component | (G) | Dimethicone * 13 | 2.00 | 2.00 | 2.00 | 2.00 | 2.00 | 2.00 |
| | | | Amodimethicone *14 | 0.20 | 0.20 | 0.20 | 0.20 | 0.20 | 0.20 |
| | | | Lanolin fatty acid * 15 | 1.00 | 1.00 | 1.00 | 1.00 | 1.00 | 1.00 |
| Total | | | | 100.00 | 100.00 | 100.00 | 100.00 | 100.00 | 100.00 |
| Component blending amount in mixture A or A' (Active ingredient concentration) (% by mass) | | | Component (A) | 0.20 | 0.20 | 0.20 | 0.00 | 1.00 | 0.20 |
| | | | Component (B) | 0.12 | 0.12 | 0.00 | 0.12 | 0.58 | 0.12 |
| | | | Component (C) | 0.28 | 0.00 | 0.28 | 0.28 | 0.28 | 0.00 |
| | | | Component (D) | 99.41 | 99.69 | 99.52 | 99.60 | 98.15 | 98.12 |
| pH of mixture A or A' | | | | - | 3.4 | - | - | - | - |

**Table 3 (cont"d.)**

| | | Comparative Example 1 | Comparative Example 2 | Comparative Example 3 | Comparative Example 4 | Comparative Example 5 | Comparative Example 6 |
|---|---|---|---|---|---|---|---|
| Production step | Mixture (A or A') prepared in step (1) | Mixing of (A), (C) and (D) | Mixing of (A), (B), and (D) | Mixing of (A), (C) and (D) | Mixing of (B), (C) and (D) | Mixing of (A), (B), (C) and (D) | Mixing of (A), (B), (C)' and (D) |
| | Appearance of mixture (A or A') prepared in step (1) | Transparent | Aggregate and precipitate | Transparent | Transparent | Aggregate and precipitate | Transparent |
| | Step (2) | - | (2-1) | (2-1) | (2-1) | (2-1) | (2-1) |
| | Temperature at the mixing in step (2) | 56°C | 56°C | 56°C | 56°C | 56°C | 56°C |
| | Blending amount of component (A) (active ingredient concentration) (% by mass) | 0.126 | 0.126 | 0.126 | 0.000 | 0.631 | 0.126 |
| | Blending amount of component (B) (active ingredient concentration) (% by mass) | 0.074 | 0.074 | 0.000 | 0.074 | 0.369 | 0.074 |
| | Amount of component (C) to be blended in mixture A or A' (active ingredient concentration) (% by mass) | 0.18 | 0.00 | 0.18 | 0.18 | 0.18 | 0.00 |
| | Blending amount of component (C) (active ingredient concentration) (% by mass) | 0.32 | 0.32 | 0.32 | 0.32 | 0.32 | 0.32 |
| | Blending amount of component (D) (% by mass) | 87.59 | 87.59 | 87.67 | 87.90 | 86.08 | 86.59 |
| Formulation of emulsion composition | Blending amount of component (E) (active ingredient concentration) (% by mass) | 2.00 | 2.00 | 2.00 | 2.00 | 2.00 | 2.00 |
| | Blending amount of component (F) (active ingredient concentration) (% by mass) | 4.48 | 4.48 | 4.48 | 4.48 | 4.48 | 4.48 |
| | Blending amount of component (G) (active ingredient concentration) (% by mass) | 2.24 | 2.24 | 2.24 | 2.24 | 2.24 | 2.24 |
| | Blending amount of component (H) (active ingredient concentration) (% by mass) | 2.00 | 2.00 | 2.00 | 2.00 | 2.00 | 2.00 |
| | Total blending amount of components (A) + (B) (active ingredient concentration) (% by mass) | 0.20 | 0.20 | 0.13 | 0.07 | 1.00 | 0.20 |
| | Cationic charges of component (A)/Anionic charges of component (B) molar ratio | 50/50 | 50/50 | - | - | 50/50 | 50/50 |
| | Blending ratio in mixture A or A' [(A+B)/C] | - | - | - | - | 1/0.18 | - |
| Evaluation results | Emulsified state | C | B | A | C | D | D |
| | Storage stability | A | A | A | A | D | D |
| | Feel at the time of application | 1.4 | 1.6 | 2.2 | 2.8 | | |
| | Moisture resistance of hair after treatment (tress width change value after storage under high humidity condition) | 1.4 | 1.6 | 1.3 | 1.4 | * | * |

| | | | | | | | |
|---|---|---|---|---|---|---|---|
| *: In Comparative Examples 5 and 6, evaluation was not performed because the conditioner was separated. | | | | | | | |

The components described in the tables are as follows.
<Component (A): Cationic Polymer or Amphoteric Polymer>
   *1 Polyquaternium-6 (dimethyldiallylammonium polymer), "MERQUAT 100" manufactured by Lubrizol Advanced Materials, Inc., cationic charge density: 6.18 meq/g, Mw: 150,000, active ingredient amount: 41.5% by mass
   *2 Polyquaternium-39 (acrylamide-acrylic acid-dimethyldiallylammonium chloride copolymer liquid), "MERQUAT 3940 POLYMER" manufactured by Lubrizol Advanced Materials, Inc., cationic charge density: 2.97 meq/g, Mw: 150,000, active ingredient amount: 43% by mass
   *3 Polyquaternium-16 (vinylimidazolium trichloride-vinylpyrrolidone copolymer) "Luviquat FC 550" manufactured by BASF SE, cationic charge density: 3.91 meq/g, Mw: 80,000, active ingredient amount: 43% by mass
   *4 Polyquaternium-22 (dimethyldiallylammonium chloride-acrylic acid copolymer liquid), "MERQUAT 280" manufactured by Lubrizol Advanced Materials, Inc., cationic charge density: 4.99 meq/g, Mw: 450,000, active ingredient amount: 41% by mass
   *5 Polyquaternium-47 (acrylic acid-methyl acrylate-methacrylamidopropyltrimethyl ammonium chloride copolymer), "MERQUAT 2001 POLYMER" manufactured by Lubrizol Advanced Materials, Inc., cationic charge density: 3.21 meq/g, Mw: 1,300,000, active ingredient amount: 21% by mass
<Component (B): Anionic Polymer>
   *6 Sodium polyacrylate, "ACUSOL 445G polymer" manufactured by The Dow Chemical Company, anionic charge density: 10.6 meq/g, Mw: 25,000, active ingredient amount: 100% by mass
   *7 (Acrylic acid/stearyl acrylate) copolymer "SOFCARE SA-37W" manufactured by Kao Corporation, anionic charge density: 9.3 meq/g, Mw: 20,000, active ingredient amount: 10% by mass
<Component (C): Organic Acid>
   *8 Lactic acid, "PURAC ULTRAPURE 90" manufactured by PURAC Tiland Ltd., active ingredient amount: 90% by mass
<Component (E): Cationic Surfactant>
   *9 Stearoxypropyltrimethylammonium chloride, "QUARTAMIN E-80K" manufactured by Kao Corporation, active ingredient amount: 45% by mass
   *10 Stearoxypropyldimethylamine, "FARMIN DM-E80" manufactured by Kao Corporation, active ingredient amount: 90% by mass, mixture of stearyl alcohol 10% by mass
<Component (F): Higher Alcohol>
   *11 Stearyl alcohol, "KALCOL 8098" manufactured by Kao Corporation, active ingredient amount: 100% by mass
<Component (H): Aqueous Medium>
   *12 Dipropylene glycol, "DPG-RF" manufactured by ADEKA CORPORATION, active ingredient amount: 100% by mass
<Component (G): Oil Agent>
   *13 Dimethicone, "Silicone KHS-3" manufactured by Shin-Etsu Chemical Co., Ltd., active ingredient amount: 100% by mass
   *14 Amodimethicone, "Silicone SF 8457 C" manufactured by Dow Toray Co., Ltd., active ingredient amount: 100% by mass
   *15 Lanolin fatty acid, "18MEA-CL1" manufactured by Kao Corporation, active ingredient amount: 3.5% by mass

### Industrial Applicability

According to the present invention, it is possible to provide a method for producing an emulsion composition which contains a polyion complex and has high stability, and when used as a hair cosmetic composition, can improve the feel at the time of application to the hair, and can suppress the spread of the hair under high humidity conditions.

## Claims

1. A method for producing an emulsion composition comprising the following components:
component (A): one or more polymers selected from the group consisting of cationic polymers and amphoteric polymers;
component (B): an anionic polymer;
component (C): an organic acid; and
component (D): water,
the method comprising the following step (1) and the following step (2-1) or step (2-2),
wherein a total blending amount of the component (A) and the component (B) in the emulsion composition is less than 1.0% by mass based on 100% by mass of the total amount of the emulsion composition:
step (1): a step for preparing a mixture A containing the components (A) to (D);
step (2-1): a step for preparing an emulsion (I) by mixing the mixture A with an oil phase component containing an emulsion forming component;
step (2-2): a step for preparing an emulsion (II) by mixing an oil phase component containing an emulsion forming component with an aqueous phase component other than the mixture A, followed by mixing with the mixture A.

2. The method for producing an emulsion composition according to claim 1, wherein the component (c) is one or more selected from the group consisting of succinic acid, lactic acid, malic acid, glycolic acid, p-toluenesulphonic acid, and naphthalenesulphonic acid.

3. The method for producing an emulsion composition according to claim 1 or 2, wherein in the mixture A, a mass ratio [(A+B)/C] of the total blending amount of the component (A) and the component (B) to the blending amount of the component (C) is in a range of 1/0.10 to 1/20.

4. The method for producing an emulsion composition according to any one of claims 1 to 3, wherein the mixture A is in a state of a suspension.

5. The method for producing an emulsion composition according to any one of claims 1 to 4, wherein the emulsion forming component comprises component (E): a cationic surfactant, and component (F): a higher alcohol.
